# EUROPEAN PATENT SPECIFICATION

(11) **EP 0 465 204 B1**
(45) Date of publication and mention of the grant of the patent: **20.09.2000**
(21) Application number: 91305971.3
(22) Date of filing: 01.07.1991
(51) Int. Cl.: A61K 39/02

(54) **Vaccine against Lyme disease**
Impfstoff gegen die Lyme-Krankheit
Vaccin contre la maladie de lyme

(30) Priority: 06.07.1990 US 549190
(43) Date of publication of application: 08.01.1992
(60) Divisional application: 99125481.4
(73) Proprietor: AMERICAN HOME PRODUCTS CORPORATION, Madison, New Jersey 07940-0874 (US)
(72) Inventor: Acree, William Marshall, Fort Dodge, Iowa 50501 (US); Wallace, Bonnie Lee, Omaha, Nebraska 68135 (US); Chu, Hsien-Jue Steve, Fort Dodge, Iowa 50501 (US); Chavez, Lloyd George, Fort Dodge, Iowa 50501 (US); Sandblom, David Scott, Humbolt, Iowa 50548 (US)
(74) Representative: Walters, Philip Bernard William

(56) References cited:
- EP-A- 0 418 827
- WO-A-90/04411
- WO-A-91/13630
- WO-A-92/00055
- DE-C- 3 942 728
- US-A- 4 721 617

## Description

The present invention relates to a vaccine to prevent or alleviate the symptoms of Lyme disease, a method for making the vaccine and a challenge model for evaluating the vaccine.

Lyme disease is caused by the spirochete Borrelia burgdorferi which is transmitted between susceptible animals by ticks of the family Ixodes, including I. dammini, I. pacificus and I. ricinus.

For man, the disease is thought to be acquired from ticks in the nymphal stage of the tick lifecycle. Most cases of Lyme disease occur in June or July when the nymphal stage occurs. Ixodes ticks in the nymphal stage are very small, about the size of a sesame seed, and are difficult to detect. It is thought that the tick must feed on the blood of the animal host for in excess of about twelve hours in order to transmit the disease causing spirochete.

A number of domesticated animals are susceptible to the disease including dogs, cats, horses, sheep, deer, birds and cattle. This poses a risk not just to the animals themselves but to their human caretakers, since the animals serve as a reservoir of spirochetes which may be transmitted to humans. This is especially true since the present inventors and others have found that dogs can transmit the disease to kennel mates even in the absence of ticks. Accordingly, it is believed that the disease can be transmitted from domesticated animals to humans.

B. burgdorferi spirochetes are difficult to grow in vitro since they require a complex mix of nutrients similar to mixtures used in mammalian tissue culture (Barbour et al. Curr. Mictobiol. 8: 123-126. 1983). The spirochete grows slowly in culture (e.g. with a 12 to 24 hour doubling time).

The symptoms of Lyme disease vary and the disease is difficult to diagnose since the symptoms are similar to the symptoms of other diseases. Early signs of the disease may include a skin rash, fever, headache and muscle and joint pain. In the weeks to months following infection, the symptoms may include inflammation of the heart and recurrent arthritis. Later, the symptoms can include chronic arthritis and dementia.

The disease can be treated after infection with a number of antibiotics including tetracycline, penicillin and erythromycin. Nonetheless, a need exists for a prophylactic treatment that will be effective throughout a two to three month period when the ticks most aggressively transmit the disease. U.S. Patent 4,721,617 by Johnson (incorporated in its entirety herein by reference) describes a vaccine comprising inactivated burgdorferi spirochetes in a physiologically acceptable vehicle. The Johnson vaccine is disclosed as effective in protecting rodents from challenge with B. burgdorferi at thirty days post vaccinations. The Johnson vaccine is not disclosed to elicit antisera which is effective in a serum neutralization assay. Nor is the vaccine disclosed to be useful in controlling the symptoms of Lyme disease. Also, the patent does not disclose a subset of B. burgdorferi proteins that elicit antisera in a protected mammal. This subset is disclosed for the first time in the present application.

Antibodies to the B. burgdorferi antigen Osp A have been described as being effective in conferring passive immunity to a treated animal. Osp A is the major outer membrane protein of B. burgdorferi and has a molecular weight of about 31K. The gene for osp A has been isolated and expression vectors have been constructed (Howe et al., Infection Immunity 54: 207-212, 1986). Accordingly, this surface protein may prove useful in a vaccine. However, the composition of this protein is thought to vary between strains of the spirochete (Barbour et al., J. Infect. Dis., 152:478-484, 1985; Bisset et al., J. Clinical Microbiol., 25: 2296-2301, 1987) as does Osp B, another burgdorferi surface protein (Barbour et al., Infection and Immunity, 45: 94-100, 1984). The major proteins of the related spirochete Borrelia hermsii (the causative agent for relapsing fever) are also known to vary between strains (Barbour et al., J, Experimental Med. 156: 1312-1324, 1982). This vanance may inhibit cross-protection from infection by varying strains of spirochete and thus may provide a mechanism by which the spirochete evades the host animal's immune system.

It is important for evaluating vaccines to have a standard, reproducible infection with a subject pathogen in a laboratory animal. Such an infection is preferably introduced into the laboratory animal by a reproducible means. A reproducible laboratory disease of this kind is a "challenge model" with which the efficacy of a vaccine in protecting an animal from challenge with the pathogen can be tested. Preferably, the challenge model closely resembles the disease as found in nature.

Various rodent challenge models (wherein the disease is induced in rodents by injection of spirochetes) have been described for evaluating the efficacy of vaccines against Lyme disease (for instance, Schaible et al., Proc. Natl. Acad. Sci. USA, 87: 3768-3772, 1990 and U.S.P. 4,721,617). However, while bacteremia can be induced in rodents, symptoms of Lyme disease in a rodent challenge model have been reported only for chemically or genetically immuno-compromised rodents (e.g. Schaible et al.. supra).

Moreover, despite the importance of dogs as a victim, reservoir, or transmissive carrier of this disease, a canine challenge model has not been described. The lack of success of previous efforts to develope a canine challenge model is described by Appel (Compendium on Continuing Education for the Practicing Veterinarian 4(11): 888-892, 1982) and by Greene et al. (Am. J. Vet, Res., 49:752-757, 1988). At best, a sub-clinical experimental B. burgdorferi infection in dogs has been described (Burgess, Zentralblatt Fuer Bakteriologie Microbiologie und Hygiene, A 263: 49-54, 1986).

WO 94/04411 (Symbicom Aktiebolat) discloses what are described as immunologically active fractions of *Borrelia Burgdorferi*. It is stated that the fractions are substantially free from cell wall and flagellar components of *B Burgdorferi* (such as flagellin) and that they have substantially the same reactivity with sera from patients with Lyme disease as that of whole cells from *B Burgdorferi* . However, this document does not provide an enabling disclosure of a vaccine.

It is an object of the present invention to provide a vaccine comprising at least two of the major antigens of *B Burgdorferi* that elicit corresponding antisera in a susceptible mammalian host.

It is a further object of the invention to provide a vaccine which protects susceptible mammals from Lyme disease, despite strain to strain valance of B. burgdorferi spirochetes.

It is another object of the present invention to provide a challenge model which displays at least one symptom of Lyme disease using mammals that are not immuno-compromised. The challenge model can be used to evaluate the efficacy of vaccines and of other treatments for Lyme disease.

It is yet another object of the present invention to provide B. burgdorferi antigens and antibodies to B. burgdorferi that can be used for diagnostic purposes.

It is yet another object of the present invention to provide a process of producing B. burgdorferi for use in the production of Lyme disease vaccines. It is still yet another object of the present invention to provide a serum neutralizing assay by which to assess the potency of antisera against B. burgdorferi.

It has now been discovered that an animal having a serum neutralizing titer of antibodies against B. burgdorferi has antibodies to a small subset of B. burgdorferi proteins having molecular weights of 14, 17, 19, 25, 28, 31, 34, 38, 41, 44, 48, 52, 54, 58, 60, 68, 80 and 90K molecular weight (±3K) and is protected from bacteremia and other symptoms of Lyme disease after challenge with B. burgdorferi spirochetes. It is believed that a vaccine comprising at least two of these major antigens, wherein at least one of the antigens is Osp A or Osp B such that each dose of vaccine comprises antigen derived from about 10⁶ to 10¹² spirochetes, and producing antisera which has a serum neutralizing titer will be effective in protecting a susceptible mammal from Lyme disease.

It has also been discovered that an artificial model of the disease (a "challenge model") can be elicited in mammals by injecting low passage spirochetes in a manner that mimics the natural route of infection.

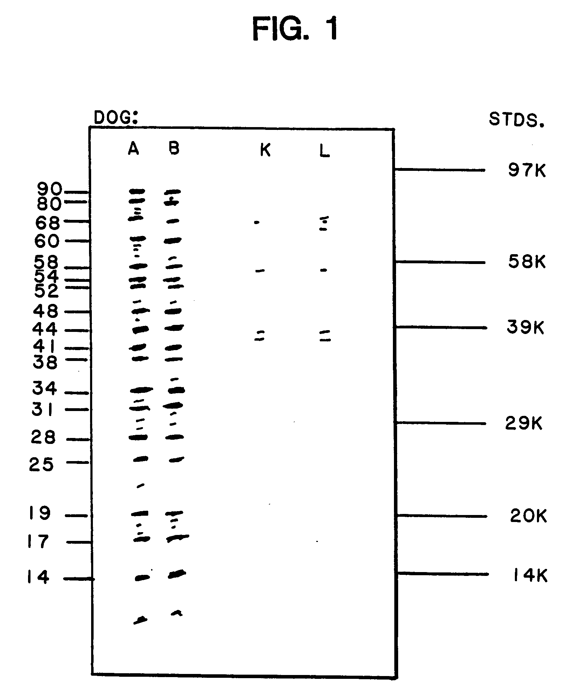

FIGURE 1. A representation of a Western blot of spirochetes vizualized using antisera from immunized dogs having serum neutralizing titers of antisera to B. burgdorferi.

### Challenge Model

Spirochetes used for challenge injections were cultured through a low number of passages after isolation from the midgut of infected ticks. Generally they are passaged no more than about 10 times. Preferably they are passaged no more than twice. A "passage" as used herein generally means an expansion in culture from about 10²-10⁴ organisms/mL to about 10⁶-10⁸ organisms/mL.

The challenge model is designed to mimic tick-borne infection as it occurs in nature. Specifically, spirochetes are repetitively injected as they would be by a feeding tick. Generally, the dose of spirochetes in any one injection is less than about 10⁸ spirochetes and preferably is between about 10 spirochetes and about 10⁶ spirochetes. In a most preferred embodiment, the number of spirochetes is between about 50 and about 50,000 spirochetes. The number of administrations is at least 2, preferably between about 4 and about 14. Preferably, at least 4 hours elapse between administrations.

Any given adminstration many also be varied to mimic natural transmission of spirochete. For instance, the spirochetes of a given administration may be injected at multiple locations on the animal. Also, at any one injection site the spirochetes can be injected at multiple depths (e.g. intramuscular (IM), intraperitoneal (IP) intradermal (ID) and subcutaneous (SC)).

The preferred schedule of spirochete administration is daily injections (approx. 1/3 IP, approx. 1/3 ID, approx. 1/3 SC) over the course of about a week.

Spirochetes can generally be recovered from challenged dogs within days of the start of challenge. Limping is generally seen after about 30 days. However, the timing of these events is expected to vary with the species of the challenged animal.

Spirochetemia can be enhanced in the challenged animal by immunosuppressing the animal by administering immunosuppressive drugs or chemicals or by irradiating with ionizing radiation. Among the suitable immunosuppressive drugs are various glucocorticoid steroids (including dexamethasone and methylprednisolone), cyclosporine, azathiopine (an analog of 6-mercaptopurine), FK-506 (Gato et al., Transplant Proc., 19(S6); 4, 1987), 15-deoxyspergualin (Todo et al., Transplant. Proc. 209(S1): 233-236, 1988), etc. Dexamethasone is available from ICN Biochemicals (Cleveland, OH) and from Schering Corp. (under the trade name Azium ∼, Kenilworth, N.J.). Methylprednisilone acetate is available as Depo-Medrol ∼ (Upjohn Co., Kalamazoo, MI) or as Depo-Predate ∼ (Legere Pharmaceuticals, Scottsdale, AZ). Dexamethasone is the preferred immunosuppressive agent according to the invention.

Spirochetemia is also expected to be enhanced by using genetically immunodeficient animals for the challenge model.

The challenge model disclosed herein can be used to test the efficacy of Lyme disease vaccines such as those disclosed below. Additionally, the challenge model can be used to test other treatments for Lyme disease including treatment with antibiotics and passive immunization.

### B. burgdorferi Vaccine Production

Preferably the B. burgdorferi strains used for the initial step in the production of vaccine retain the ability to induce spirochetemia in a susceptible mammal. B. burgdorferi strain B-31 is most preferred (available from the American Type Tissue Collection under accession No. 35210).

The B.burgdorferi spirochetes may be grown in BSK II medium (Barbour et al., Yale J. Biol. Med. 57: 521-525, 1984) or the nutritional equivalent. Since this medium is an adaptation of a culture medium designed for mammalian cells (which are much more complex), it is anticipated that numerous ingredients may be omitted without significant effect on the growth rate of B. burgdorferi. Such alterations in the growth medium are readily verified without undue experimentation and are within the scope of the present invention.

The culture temperature is generally between 25'C and 40'C, preferably between about 32'C and about 38'C most preferably 34'C. Preferably, the temperature is maintained within 1'C of the set-point temperature throughout the culturing incubation.

Generally the pH of the culture medium is between 7.0 and 8.0. Preferably the pH is maintained within ±0.2 pH units of the desired pH over the course of the culturing incubation by the addition of acid or base as appropriate. Most preferably, the pH is 7.6 ±0.2.

The culturing incubations were generally in a vessel which was agitated to accelerate the exchange of gases between the culture and the air. Preferably the agitation is done with an impeller rotated at between about 25 and about 100 rpm.

Significantly, the course of the culturing incubation is monitored by assessing the level of production of one of the B. burgdorferi major surface proteins, Osp A or Osp B. Methods for monitoring these proteins are described in Example 2. The B. burgdorferi cultures are stopped when a maximum level of one of these proteins is achieved. For the protein being monitored, it is preferred that at least 60% of the B. burgdorferi cells present at the end of the culturing incubation express the protein. This can be measured by an immunofluorescence assay (described in Example 1). More preferably, at least 70% of the organisms express the protein. Still more preferably, at least 90% of the organisms express the protein.

In one embodiment of the invention, after the culturing incubation, the organisms are inactivated or antigenic fractions are collected free of live or virulent spirochetes. The organisms can be inactivated by a number of chemical agents including merthiolate, beta-propylene lactone, binary ethylene amine, phenol, glutaraldehyde and formaldehyde. The organisms can also be inactivated with heat (e.g. 60'C for several hours).

Preferred inactivators are aldehydes such as glutaraldehyde or formaldehyde. Formaldehyde is most preferred. Preferably the concentration of aldehyde used is between about 0.05% and about 1% v/v. Most preferably, the concentration is 0.1%.

Antigenic fraction can be collected, for example, by gel filtration, electrophoresis, affinity chromatography, and/or centrifugation.

Prior to administration to a mammal to be treated, the harvested antigens and/or spirochetes are suspended in a physiologically acceptable carrier. Preferably, the antigens/spirochetes will be admixed with an adjuvant such as aluminum hydroxide, aluminum phosphate, saponin, oil, or a polymer known to have adjuvant activity.

A number of acrylic acid polymers and copolymers of acrylic acid and methacrylic acid and styrene have adjuvant activity. Polyvinyl Chemical Industries (Wilmington, Mass.) sells such polymers under the tradename NEOCRYL. NEOCRYL A640, an aqueous acrylic copolymer having pH 7.5, viscosity 100eps (Brookfield 25'C), a weight per litre of 0.86 kg (weight per gallon of 8.6 pounds) as supplied containing 40% solids by weight, 38% solids by volume and an acid number of 48, is a preferred adjuvant. Specifically, NEOCRYL A640 is an uncoalesced aqueous acrylic copolymer with styrene. More specifically, NEOCRYL A640 is a latex emulsion of a copolymer of styrene with a mixture of acrylic and methacrylic acid. Other useful NEOCRYL grades are 520 and 625, and NEOREZ 966. The term "CSMA" will be used hereinafter to refer to a copolymer of styrene and a mixture of acrylic acid and methacrylic acid.

Ethylene/maleic anhydride copolymer is another preferred adjuvant. Suitable grades of ethylene/maleic anhydride copolymer useful in this invention are the linear ethylene/maleic copolymers such as EMA-31 (as produced by Monsanto Co., St. Louis, Missouri), a copolymer with approximately equal amounts of ethylene and maleic anhydride, having an estimated average molecular weight of about 75,000 to 100,000. These copolymers are water soluble, white, free-flowing powders having the following typical properties: a true density of about 1.54 g/mL, a softening point of about 170'C, a melting point of about 235'C, a decomposition temperature of about 274'C, a bulk density of about 320.8 kg/m³ (20 lbs/ft³) and a pH (1% solution) of 2.3.

More preferably, two or more adjuvants will be admixed with the harvested antigens and/or spirochetes. The preferred combination is ethylene/maleic anhydride copolymer and NEOCRYL A640.

The pH of the vaccine is generally adjusted to between about pH 6.8 and about pH 7.7 by addition of 1N acid or base as appropriate.

The harvested antigens and/or spirochetes may be conjugated with an immuno-stimulating macromolecule such as keyhole limpet hemocyanin or tetanus toxin. Conjugation of proteins to macromolecules is disclosed by Likhite (U.S.P. 4,372,945) and by Armor et al. (U.S.P. 4,474,757). General aspects of vaccine preparation are described in New Trends and Developments in Vaccines (Voller et al., ed., University Park Press, Baltimore, MD., 1978).

The antigens can be collected free of live or virulent organisms by several methods including fragmentation techniques, such as gel filtration, immuno-affinity chromatography using antisera disclosed herein, differential centrifugation, or electrophoresis. For instance, it has been discovered that the outer membrane antigens of B. burgdorferi can be extracted with low concentrations of either ionic or monionic detergents. These extracted antigens are effective to elicit an immune response in a vaccinated animal.

Preferred detergents for this embodiment of the invention are sodium dodecyl sulfate (SDS) and Triton™ X100 (available from Sigma Chemical, St. Louis, Missouri, and others).

The extraction protocol begins with B. burgdorferi organisms, preferably at a concentration of between about 10⁸ organisms/mL, more preferably between about 10⁹ organisms/mL and about 10¹² organisms/mL. Detergent is then added, preferably to a concentration between about 0.2% weight/volume and about 0.6% w/v for an ionic detergent such as SDS. For nonionic detergents the concentration is preferably between about 0.25% w/v and about 2.0% w/v, and more preferably between about 0.8% w/v and about 1.2% w/v.

The organisms are then agitated, generally at between about 100 rpm and about 300 rpm for between about 0.5 hours and about 10 hours. Preferably the agitation is maintained for between about 1 and 2 hours.

The outer membrane proteins are then removed from the major portion of the bacteria by centrifugation. After centrifugation the supernate is enriched in outer membrane protein and the pellet contains the organisms.

When applied to B. burgdorferi the procedure extracts the major B. burgdorferi antigens and provides antigens for a vaccine which protects susceptible mammals from fever caused by B. burgdorferi.

The vaccine is prepared from the extracted antigens by the methods described above.

In another embodiment of the invention, after the culturing incubation the spirochetes are processed to isolate specific major antigens of B. burgdorferi.

It has been found that a vaccine according to the invention induces antisera to a plurality of B. burgdorferi by antigens having the following molecular weights: 14, 17, 19, 25, 28, 31, 34, 38, 41, 44, 48, 52, 54, 58, 60, 68, 80 and 90K ± 3K). These antigens are designated herein as "major antigens" of B. burgdorferi or their biological equivalents. According to the invention, an effective vaccine will induce antisera to at least two of the major antigens (at least one of which is Osp A or Osp B). The known variability of at least two B. burgdorferi antigens suggest that a monospecific vaccine may not be effective against a broad spectrum of burgdorferi isolates.

More preferably, the vaccine is effective to induce antisera against at least three of the major antigens. One of the antigens included in the vaccine is Osp A, (MW 31K) or Osp B (MW 34K). The 31, 34 and 41k proteins are the Osp A, Osp B and flagellate proteins of B. burgdorferi, respectively.

The serum neutralization assay described in Example 3 can be used to assess the relative potency of antisera directed against a given B. burgdorferi antigen. Potency of the antisera against B. burgdorferi infection is expected to correlate with the ability of the antisera to neutralize B. burgdorferi in the serum neutralization assay. The assay can also be used to assess whether antisera directed against two or more of the antigens is more effective than monospecific antisera. It is anticipated that even if antiserum which is monospecific for a given antigen is by itself ineffective in the serum neutralization assay, the antisera may enhance the potency of another monospecific antisera. Accordingly, the serum neutralization assay can be used to validate a vaccine which elicits antisera directed against less than the full complement of major antigens.

Thus, antisera directed against each of the disclosed major antigens can be manufactured. For instance, the antigens of an extract of whole spirochetes can be separated by SDS polyacrylamide gel electrophoresis and the specific bands excised. The location of these bands can be confirmed by electroblotting a portion of the polyacrylamide gel and assaying the blot by standard Western blotting techniques (see Molecular Clonning, 2nd edition, Sambrook et al. eds., Cold Spring Harbor Press, 1989 and Example 5 below).

The antigen-containing gel slices can be used to raise antigen specific antisera by several methods. For example, the gel slice can be broken up by a method such as extrusion through a narrow gauge needle. The pulverized gel can then be injected into the animal which is to produce antisera. Adjuvants may be added to the injection material although the polyacrylamide gel material is anticipated to act as a polymer type adjuvant. Alternately, the antigen may be eluted from the gel slice by a number of known methods including incubating the gel slice in an aqueous detergent solution until the antigen is substantially eluted from the gel slice. Or, the antigens can be electroeluted from the gel slice. Devices for electroelution are available from several manufacturers Schleicher & Schuell (Keene, NH) and Bio-Rad Labs (Richmond, CA). Such devices can also be produced from common laboratory equipment (see Molecular Cloning, supra, pp. 6.28-6.29). The eluted antigens can then be administered, with or without an adjuvant, to the animal which is to produce antisera.

In another method, the antigens are separated by SDS polyacrylamide gel electrophoresis and transferred to one of various adsorptive membranes in the same way as is done in Western blotting. The specific antigens can then be isolated by excising the appropriate region of the transfer membrane. Suitable membranes include the nitrocellulose membranes commonly used in Western blotting, Immobilon∼-P from Millipore Corp. (Bedford, MA), and glass fibre membranes which are coated with polyamines (Vandekerckhove et al. Eur. J. Biochem. 152: 9-19,1985 and Abersold et al., J. Biol. Chem. 261: 4229-4238, 1986). The antigen-containing membrane slices can be surgically implanted subcutaneously in the animal which is to produce antibody. The membrane is anticipated to behave as a slow release adjuvant.

The major antigens are known to be effective in eliciting an antibody response (see Example 5). Some of these antigens are believed to elicit a T-killer cell response. These can be found by isolating lymphocytes from immunized animals and assaying T-cell responses to specific major antigens of B. burgdorferi. T-cell activation responses include an increase in intracellular calcium, production of lymphokines, cell proliferation, and cytolytic activity. Such in vitro measurements of T-cells are described by Weis, et al. (Adv. Immunol. 41: 1-38, 1987,).

B cells from antisera producing animals can be used for the isolation of monoclonal antibodies ("mcAb") using known techniques (Campbell, Monoclonal Antibody Technology, Vol. 13, Elsevier, Amsterdam 1984; Harlow et al., Antibodies: A Laboratory Manual, Cold Spring Harbor Press, New York, 1988; Goding, Monoclonal Antibodies: Principle and Practice, Academic Press, London, 1986).

The electrophoretic migration of the individual major antigens is anticipated to vary somewhat between isolates of B. burgdorferi. The Western blot procedure of Example 5 may be used to identify the SDS polyacrylamide gel electrophoresis migration pattern for other B. burgdorferi isolates. The immunochemical equivalence of a variant to one of the major antigens disclosed herein can be established by Western blot analysis using monospecific antisera to visualize the bands. Electrophoretic variants of individual B. burgdorferi antigens can be isolated and antisera raised against them can be evaluated for relative effectiveness in the serum neutralization assay.

As described in detail below under the heading "Diagnostics', the disclosures of the present application are sufficient to teach the isolation and sequence characterization of the major antigens of B. burgdorferi. Therefore, vaccines composed of synthetic antigens or the biological equivalents are within the scope of the present invention.

Accordingly, in a further embodiment of the vaccine, the vaccine comprises at least two of the major antigens of B. burgdorferi (at least one of which is Osp A or Osp B) or their biological equivalents (e.g., synthetic proteins). The vaccine is combined with a physiologically acceptable carrier and optionally adjuvanted as described above.

In a still further embodiment of the vaccine, the vaccine is characterized by being effective to protect a susceptible mammal from fever, or arthritis.

It will be understood by the skilled practitioner that the vaccines of the present invention may be combined with other vaccines to produce a combination vaccine effective against more than one pathogen. Examples include a Lyme disease vaccine in combination with one or more of vaccines for Leptospira canicola, Leptospira icterohaemorrhagiae, Leptospira hardjo, Leptospira grippotyphasa, feline leukemia, rhinotracheitis, calici, panleukopenia, chlamydia psittaci, canine distemper, adenovirus Type 2, coronavirus parainfluenza, and parvovirus.

### Vaccination

The vaccine is preferably administered by injection either subcutaneously, intradermally, intraperitoneally or intramuscularly. Intramuscular injection is preferred. The vaccine may be administered by single or by multiple injections. Since oral exposure to B. burgdorferi has been found to elicit an antibody response in cats (Kimminan, Veterinary Technician, 10:385-397, 1989), an oral vaccine is also contemplated and within the scope of the present application.

The amount of spirochete antigens present in each vaccine dose is selected to be at least the amount sufficient to induce a serum neutralizing titer of antibodies. (The serum neutralization assay measures the ability of antisera in conjunction with complement to neutralize B. burgdorferi and is described in more detail in Example 3.) Each dose will comprise antigen derived from about 10⁶ to about 10¹² spirochetes, preferably from between about 10¹⁰ to about 10⁹ spirochetes. An optimum amount of antigenic product for a particular production of vaccine can be ascertained by standard studies which monitor the serum neutralization titers of vaccinated mammals.

### Diagnostics

Monospecific antisera to several of the major antigens (MWs 14, 17, 19, 25, 28, 38, 44, 48, 52, 54, 58, 60, 68, 80 and 90K ±3K) may be used in diagnostic kits used to assay body fluids or tissue extracts for the presence of B. burgdorferi antigens. Techniques for using these antibodies as probes for antigens are discussed above and by Harlow et al. (Antibodies: A Laboratory Manual, Cold Spring Harbor Press, New York, 1988).

Additionally, the individual antigenic proteins can be used as diagnostic aids. The protein or a mix of the major antigens isolated as described above can be separated by sodium dodecyl sulfate-polyacrylamide gel electrophoresis (SDS-PAGE). The gel can be electroblotted onto a membrane. Then, a strip of the membrane can be developed as a "Western blot" using serum from an animal to be diagnosed. As controls, known positive or negative sera can be used to visualize equivalent membrane strips.

Genomic and subgenomic DNA can be isolated from B. burgdorferi and the DNA can be fragmented with a restriction enzyme. The digested DNA can then be ligated into a plasmid or phage expression vector. Colonies or plaques (as appropriate) can be grown-up and transferred in-part to a membrane which can be assayed using antisera against one or more of the major antigens. Positive plaques or colonies can be expanded and the proteins they express (generally after the vector-expressed protein is induced with a suitable agent, usually isopropylthio-beta-D-galactoside) can be separated by SDS-PAGE. The proteins can, thereby, be studied by the Western blot techniques discussed above.

Accordingly, the DNA encoding the major antigens, DNA hybridizing to this DNA under stringent conditions, and DNA encoding substantially the same protein are within the scope of the present invention.

The invention is further illustrated by the following nonlimiting examples.

### Example 1 - The Challenge Model

### Dogs

Twenty-six (26) beagles (from Theracon, Inc. - Topeka, KS), which were 16 to 18 weeks old and had no previous history of exposure to B. burgdorferi, were used. Four challenge groups of five dogs per group were evaluated. Groups 1 and 2 and three contact controls were housed together in room A. Groups 3 and 4 and three contact controls were housed together in room B.

### B. burgdorferi Isolate from Ticks

Ticks infected with B. burgdorferi were collected in Wisconsin and the alimentary tracts (midgut) were removed from approximately 1000 ticks and emulsified in Barbour-Stoenner-Kelly (BSK) medium (Barbour et al. Curr. Microbiol. 8: 123-126, 1983) and stored at -100'C in vials containing 2 mL aliquots. Before use, this material was thawed, polled, and concentrated by centrifugation to 10,000 microorganisms per 1 mL aliquot.

### Challenge Schedule

The dogs in each of the four groups received seven consecutive daily doses of spirochete challenge at different sites on the abdomen. Each dose contained 500 microorganisms per 0.5 mL. The challenge was delivered using a tuberculin syringue by releasing material intraperitoneally (IP), intradermally (ID) and subcutaneously (SC) as the needle was withdrawn from the infection site.

### Immunosuppression

Dogs in Group 1 were given a one mL does of dexamethasone (2 mg/mL, Azium ∼, Schering Corp., Kenilworth, NJ 07033) on the day of challenge and days 2, 4, and 6 following initiation. Dogs in Group 2 were not treated with an immunosuppressant during the challenge period. Dogs in Group 3 were given a one mL doses of methylprednisolone acetate (20 mg/mL, Depo-Medrol∼, UpJohn Co., Kalamazoo, MI 49001) on the first day of challenge ("doc") and on the 6th day post-challenge ("dpc", measured from day of challenge). Dogs in Group 4 did not receive an immunosupressant during the challenge period.

Sixty days post challenge, all dogs in Groups 1 and 2 and the contact controls received a one mL dose of dexamethasone; all dogs in Groups 3 and 4 and the contact controls received a one mL dose of methylprednisolone acetate.

### Examination of Blood Samples for B. burgdorferi

Following the first dose of the spirochete challenge, blood samples were taken from all 26 dogs twice daily for 14 consecutive days. At 60 days post challenge blood samples were taken twice daily for 10 consecutive days and blood samples were taken at the time of necropsy (90 days post challenge). Blood was collected in tubes containing a 15% solution of Na₂ EDTA (ethylene diamine tetraacetic acid).

One mL of whole blood was added to 6 mL of BSK medium and the mixture was incubated at 32'C for six weeks. Cultures were examined using an indirect fluorescent antibody (IFA) method. The cultures were prepared on slides, allowed to air dry and fixed in cold acetone. Slides were flooded for 30 minutes at 37'C with a dilution of a murine antibody (IgG isotype) directed against B. burgdorferi or a given B. burgdorferi antigen. The slides were then rinsed with phosphate buffer saline (PBS). A dilution of fluorescein isothiocyanate (FITC) conjugated anti-IgG mouse sera (Jackson Immuno Research labs, West Grove, PA) was then added to each slide and incubated at 37'C for another 30 minutes. Slides were washed in PBS and read microscopically using a fluorescence microscope. The presence of fluorescent spirochetes indicates a positive isolation.

Antibodies for use in the IFA assay include polyclonal antisera against B. burgdorferi (sheep antibodies are available from Fitzgerald Industries International, Action, Mass.) and monoclonal antibodies may also be used (e.g. Cb-2, a mouse monoclonal antibody to Osp B is available from Veterinary Clinical Resources, P.O. Box 969, Elgin, Tx). Additionally, antisera produced by the methods taught in the present application may be used.

### Antibody Titers

Dogs were bled at the time of challenge and then at 2, 4, 6, 8, 10, 14, 30, 60 and 90 dpc. A standard suspension of B. burgdorfei (2 X 10⁶ organisms per mL) was placed on a slide and fixed with cold acetone for 60 minutes. Two-fold serial dilutions of test sera were incubated on the slide at 36'C for 30 minutes before they were thoroughly rinsed with phosphate-buffered saline ("PBS"). FITC-labeled anti-dog IgG (from goat) was then added to each slide and incubated at 36'C for an additional 30 minutes. Slides were washed with PBS and the results determined by fluorescence microscopy. The endpoint of the IFA antibody titer was determined as the highest serum dilution which showed positive fluorescence.

### Results

Early recovery of spirochetes from blood (0-14 dpc) is shown in Tables 1 and 2. The recovery of spirochetes over the course of the treatment regimen is summarized in Table 3. One conclusion is that spirochetes are recovered from all challenged dogs within four days. For immunosuppressed dogs, spirochetes are recovered within about two days.

Surprisingly, after a longer delay spirochetes are recovered from the contact control dogs which were not challenged with spirochetes. However, the degree of spirochetemia appears to be lower than for treated dogs since the frequency at which spirochetes are recovered is lower.

Antibody titers (measured by IFA) for serum drawn at the designated times are shown in Tables 4 and 5. The high background titers for dogs not exposed to spirochetes (1:16 to 1:128) have been observed by others. The conclusion from the data in these tables is that immunosuppressive drugs enhance the antibody response to B. burgdorferi spirochetemia. Presumably, this enhancement is due to greater levels of spirochetemia obtained when the animal is immunosuppressed.

Additionally, limping was observed in 75% of the challenge dogs. Sixty to 80% of the dogs in each challenge group were observed to limp, while none of the contact control dogs were observed limping.

The present challenge model for Lyme disease is especially advantageous in that symptoms of Lyme disease are displayed in the challenged animals even when they are not immunosuppressed. This suggests that the disease displayed in the laboratory situation is more like that found in animals infected naturally.

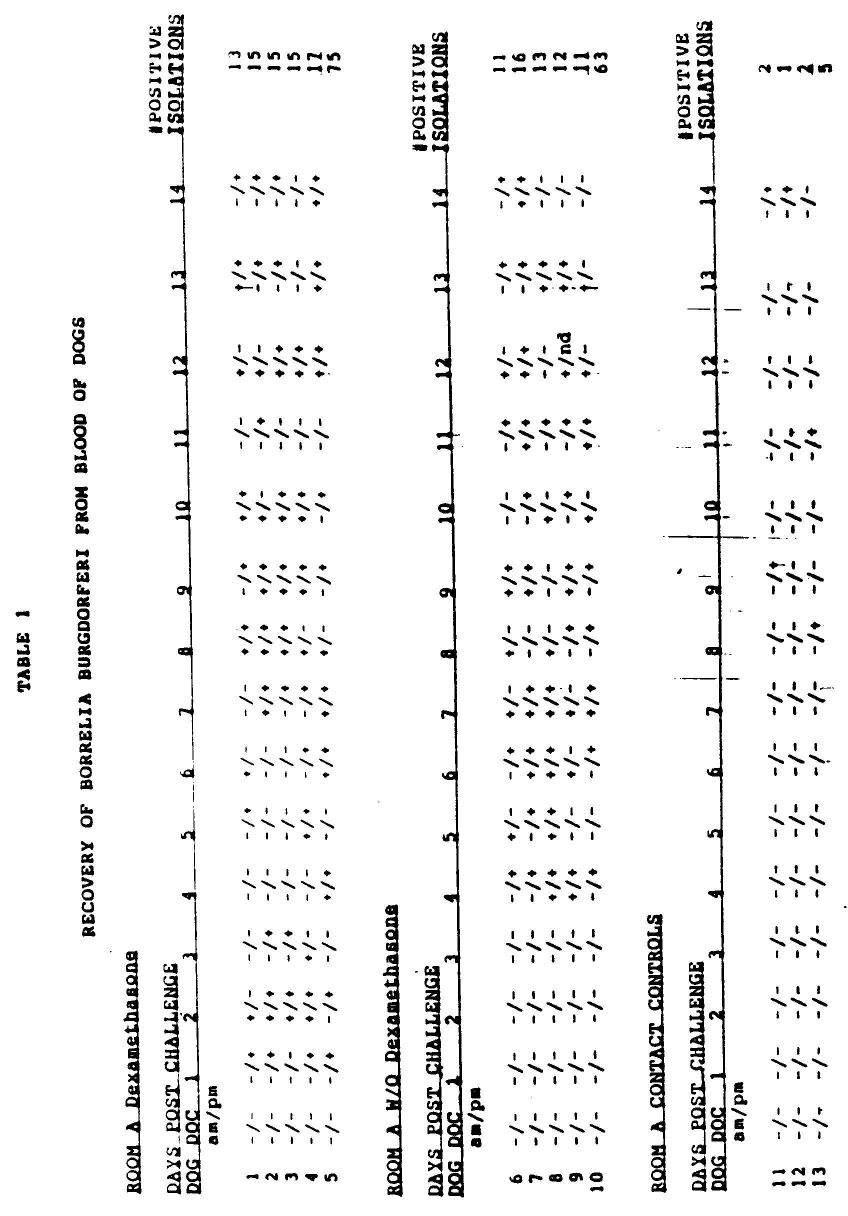

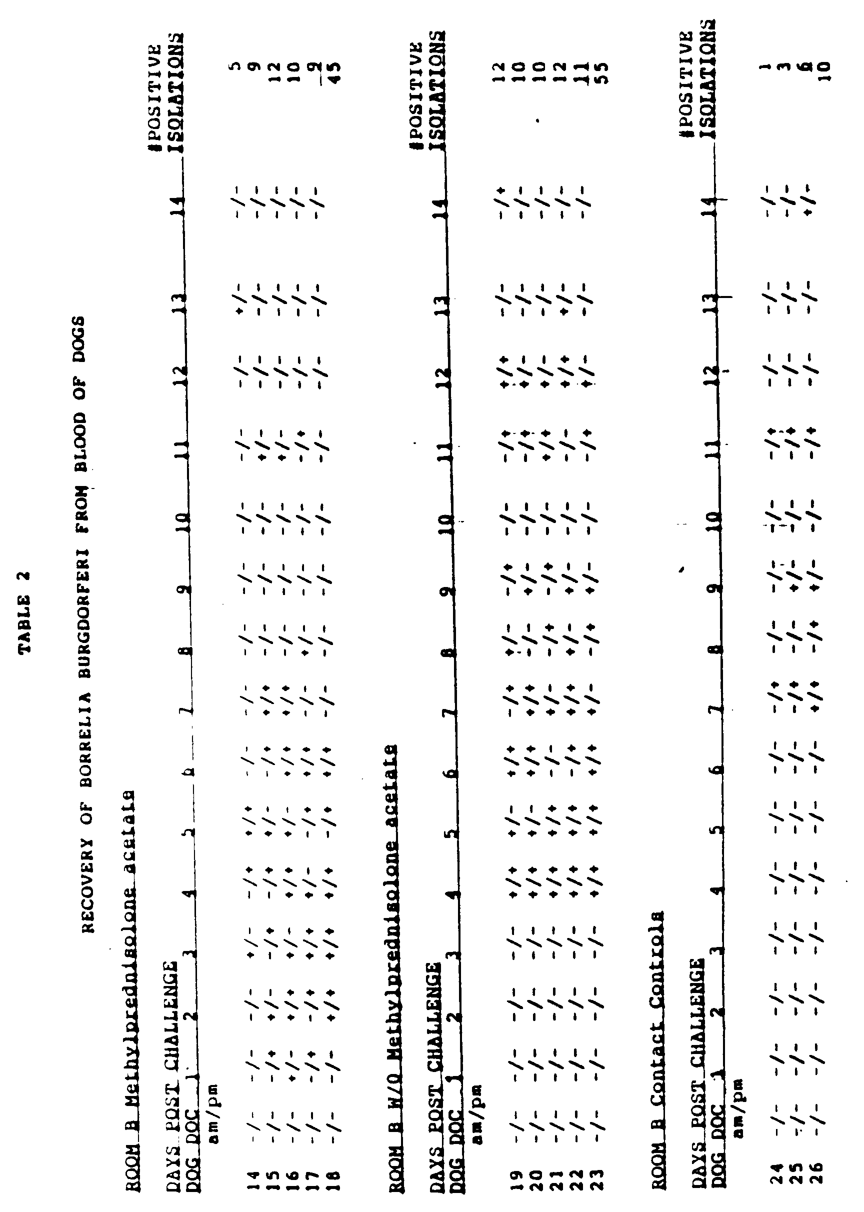

**TABLE 3**

| PERCENTAGE OF BLOOD SAMPLES GIVING POSITIVE RECOVERY | | | | |
|---|---|---|---|---|
| Time DPC | Dexamethasone Treated | Methyprednisolone Treated | No drug at Challenge¹ | Contact Control² |
| 0-14 | 50% | 32% | 42% | 9% |
| 60 | 80% | 0% | 50% | 33% |
| 61-63 | 53% | 53% | 40% | 61% |
| 90 | 80% | 60% | 50% | 50% |
| All dogs were immunosuppressed on day 60. | | | | |

| | | | | |
|---|---|---|---|---|
| ¹Observations for dogs from Rooms A and B (Groups 2 and 4) | | | | |
| ²Observations for contact control dogs from Rooms A and B | | | | |

**TABLE 4**

| RECIPROCAL IFA ANTIBODY TITERS TO BORRELIA BURGDORFERI ROOM A Dexamethasone DAYS POST CHALLENGE: | | | | | | | | | | |
|---|---|---|---|---|---|---|---|---|---|---|
| DOG | DOC | 2 | 4 | 6 | 8 | 10 | 14 | 30 | 60¹ | 90 |
| 1 | 64 | 64 | 64 | 128 | 128 | 256 | 128 | 102400 | 102400 | 102400 |
| 2 | 64 | 64 | 64 | 64 | 64 | 128 | 128 | 1600 | 102400 | 408600 |
| 3 | 64 | 128 | 128 | 256 | 512 | 512 | 512 | 25600 | 102400 | 102400 |
| 4 | 64 | 64 | 64 | 128 | 128 | 128 | 64 | 1600 | 102400 | 1638400 |
| 5 | 128 | 128 | 128 | 256 | 256 | 256 | 256 | 25600 | 102400 | 409600 |
| GMT² | 73 | 84 | 84 | 147 | 169 | 223 | 147 | 11143 | 102400 | 310267 |

| ROOM A W/O Dexamethasone DAYS POST CHALLENGE: | | | | | | | | | | |
|---|---|---|---|---|---|---|---|---|---|---|
| DOG | DOC | 2 | 4 | 6 | 8 | 10 | 14 | 30 | 60¹ | 90 |
| 6 | 16 | 64 | 64 | 128 | 64 | 128 | 128 | 6400 | 6400 | 6400 |
| 7 | 32 | 64 | 512 | 512 | 512 | 512 | 512 | 6400 | 25600 | 102400 |
| 8 | 128 | 256 | 256 | 256 | 256 | 256 | 256 | 1600 | 25600 | 25600 |
| 9 | 128 | 256 | 256 | 512 | 512 | 512 | 512 | 1600 | 102400 | 102400 |
| 10 | 64 | 64 | 128 | 256 | 256 | 256 | 256 | 400 | 1600 | 6400 |
| GMT | 56 | 111 | 194 | 294 | 256 | 294 | 294 | 2111 | 14703 | 25600 |

| ROOM A Contact Controls DAYS POST CHALLENGE: | | | | | | | | | | |
|---|---|---|---|---|---|---|---|---|---|---|
| DOG | DOC | 2 | 4 | 6 | 8 | 10 | 14 | 30 | 60¹ | 90 |
| 11 | 16 | 16 | 32 | 32 | 32 | 16 | 32 | 32 | 128 | 128 |
| 12 | 128 | 128 | 128 | 256 | 256 | 256 | 256 | 256 | 256 | 256 |
| 13 | 128 | 512 | 512 | 512 | 1024 | 1024 | 1024 | 1024 | 1024 | 1024 |
| GMT | 64 | 102 | 128 | 161 | 203 | 161 | 203 | 203 | 322 | 322 |

| | | | | | | | | | | |
|---|---|---|---|---|---|---|---|---|---|---|
| 1 All dogs were immunosuppressed with dexamethasone at 60 dpc. | | | | | | | | | | |

**TABLE 5**

| IFA ANTIBODY TITERS to BORRELIA BURGDORFERI ROOM B Methylprednisilone DAYS POST CHALLENGE: | | | | | | | | | | |
|---|---|---|---|---|---|---|---|---|---|---|
| DOG | DOC | 2 | 4 | 6 | 8 | 10 | 14d | 30 | 60¹ | 90 |
| 14 | 32 | 64 | 256 | 128 | 256 | 256 | 1024 | 102400 | 409600 | 1638400 |
| 15 | 32 | 128 | 128 | 256 | 256 | 256 | 256 | 6400 | 25600 | 409600 |
| 16 | 16 | 64 | 64 | 64 | 64 | 128 | 128 | 102400 | 409600 | 1638400 |
| 17 | 128 | 256 | 512 | 512 | 512 | 512 | 1024 | 1600 | 409600 | 1638400 |
| 18 | 32 | 128 | 128 | 512 | 512 | 1024 | 1024 | 25600 | 409600 | 1638400 |
| GMT | 37 | 111 | 169 | 223 | 256 | 338 | 512 | 19401 | 235253 | 1241675 |

| ROOM B W/O Methylprednisilone DAYS POST CHALLENGE: | | | | | | | | | | |
|---|---|---|---|---|---|---|---|---|---|---|
| DOG | DOC | 2 | 4 | 6 | 8 | 10 | 14d | 30 | 60¹ | 90 |
| 19 | 32 | 128 | 128 | 128 | 128 | 256 | 512 | 600 | 25600 | 25600 |
| 20 | 32 | 64 | 64 | 128 | 128 | 256 | 256 | 600 | 25600 | 409600 |
| 21 | 32 | 128 | 128 | 128 | 128 | 256 | 256 | 1600 | 102400 | 409600 |
| 22 | 128 | 256 | 256 | 128 | 256 | 256 | 128 | 6400 | 25600 | 102400 |
| 23 | 32 | 64 | 64 | 64 | 64 | 64 | 64 | 400 | 25600 | 102400 |
| GMT | 42 | 111 | 111 | 111 | 128 | 194 | 194 | 1081 | 33779 | 135118 |

| ROOM 3 Contact Controls DAYS POST CHALLENGE: | | | | | | | | | | |
|---|---|---|---|---|---|---|---|---|---|---|
| DOG | DOC | 2 | 4 | 6 | 8 | 10 | 14 | 30 | 60¹ | 90 |
| 24 | 64 | 128 | 128 | 128 | 128 | 128 | 128 | 128 | 128 | 256 |
| 25 | 128 | 512 | 512 | 512 | 512 | 512 | 512 | 512 | 1024 | 1024 |
| 26 | 128 | 256 | 256 | 512 | 512 | 256 | 256 | 256 | 512 | 512 |
| GMT | 102 | 256 | 256 | 322 | 322 | 322 | 256 | 256 | 406 | 406 |

| | | | | | | | | | | |
|---|---|---|---|---|---|---|---|---|---|---|
| 1 All dogs were immunosuppressed with methylprednisilone at 60 dpc. | | | | | | | | | | |

### Example 2 - B. burgdorferi Vaccine Production

### B. burgdorferi Strains

B. burgdorferi strain B-31 (available from the American Type Tissue Collection under accession No. 35210) was used to seed large-scale antigen-producing cultures.

Other B. burgdorferi strains in culture are anticipated to be useful in the production of B. burgdorferi antigens. Preferably, strains used in the production of vaccine are able to cause spirochetemia in a mammal.

### Spirochete Growth Medium

The growth medium is prepared by modifying the BS II formula described by Barbour et al. (Yale J. Biol. Med. 57: 521-525, 1984). The formula used is shown below:

| Formula | |
|---|---|
| Substance | Amount |
| CMRL 1066¹ (10X) (GIBCO, Grand Island, NY) | 83 mL/L |
| Neopeptone² (DIFCO, Detroit, MI) | 4.6 g/L |
| Sodium Bicarbonate (U.S.P.) | 1.8 g/L |
| Yeast Extract (Oxoid USA, Columbia, MD) | 3.3 g/L |
| HEPES buffer (acid form) (Research Organics, Cleveland, OH) | 5.7 g/L |
| Glucose (U.S.P.) | 4.3 g/L |
| Citric Acid - Tripotassium salt (SIGMA Chemical, St. Louis, MO) | 1.0 g/L |
| Sodium Pyruvate (Sigma) | 0.7 g/L |
| N-Acetyl-D-Glucosamine (Sigma) | 0.5 g/L |
| L-Glutamine³ (GIBCO) | 0.4 g/L |
| Bovine Serum Albumin³ (Intergen Biochem., Tuckahoe, NY) | 25 g/L |
| Fetal Calf Serum³ (HAZLETON, Lenexa, KS) | 4.2 mL/L |
| Deionized Water | to volume |

| | |
|---|---|
| ¹ Described by Parker et al. Special Publications of the New York Academy of Science Vol. 5, p. 303, 1957, incorporated herein by reference. | |
| ² Beef protein hydrozylate. | |
| ³ Heat labile. | |

Gently heat a volume of deionized water (approx. 80% of final volume) to a temperature which facilitates the dissolution of the above-listed solids (e.g. 30'C to 40'C). Add the above-listed ingredients and stir until all solids dissolve. The pH is then adjusted to 7.6 ± 0.1 with a concentrated aqueous solution of NaOH. The volume is adjusted to the final volume with deionized water. The solution is then sterile filtered.

Alternately, a solution of the listed components minus the heat labile components is made in a reduced volume and sterilized by autoclaving. Filter-sterilized concentrated solutions of the heat-labile components are then added and the volume adjusted with sterile water.

The manufacturers listed as the sources of the medium components are not meant to be restrictive but rather to be illustrative of acceptable quality. The components of the medium are well known in bacteriology and/or mammalian tissue culture and acceptable materials are available from other manufacturers.

It has been found that the gelatin component described by Barbour et al. is not required for B. burgdorferi growth. In fact, the gelatin interferes in processing B. burgdorferi by increasing the viscosity of the bacterial culture. The presence of gelatin also precludes storing a culture in a refrigerator prior to processing since the culture "gels" at low temperature. Gelatin is optionally added (at 11.7 g/L) to medium used in intermediate and small scale cultures (25 mL to about 10L) but is generally not added to medium used for production-scale cultures (10L and more).

### Growth of B. burgdorferi

The bacteria are grown in smaller vessels until sufficient bacteria are present to inoculate a large scale fermentation vessel (preferably 800 to 2000L). Cultures are generally inoculated with 3 x 10⁵ organisms per mL of medium.

The fermentation is conducted with the following parameters monitored and controlled:

| | |
|---|---|
| Temperature | 34'C ± 1'C |
| pH | 7.6 ± 0.2 |
| Impeller RPM | 25 - 100 |

Generally the culture is grown (for approximately 6 days) until the concentration of organisms is at least 1 x 10⁸/mL. However, it is important for large scale production purposes to monitor the production of B. burgdorferi major surface proteins over the course of the fermentation. The culture is stopped at the time when the concentrations of either Osp A or Osp B substantially reaches the maximum (beyond which time the concentration declines).

### Monitoring Antigens

The antigen levels can be measured by a number of techniques including SDS polyacrylamide gel electrophoresis (SDS-PAGE), Western blots using antisera to B. burgdorferi antigens, other antibody-mediated assays including RIA, ELISA and complement fixation (see Fundamental Immunology, W.E. Paul, ed., Raven Press, New York, 1989, pp. 315-358, incorporated herein by reference).

For this example, the antigens are monitored with the IFA assay outlined in Example 1. Additionally, the number of cells expressing one of the major B. burgdorferi surface proteins, Osp A or Osp B, is monitored with this assay.

### Harvest and Purification

Once the maximum concentration of Osp A or Osp B is achieved, the culture (in this embodiment) is inactivated by adding formaldehyde to a concentration of 0.1%. The inactivated spirochetes are then concentrated by removing the growth media either by centrifugation, hollow fiber filtration or tangential flow filtration. The inactivated organisms are optionally washed with water or a balanced salt solution. Finally, the inactivated organisms are resuspended in water or balanced salt solution to form a cell concentrate having ≥ 5 x 10⁸ organisms per mL.

### Vaccine Formulation

The concentrate is diluted to a concentration of 5 x 10⁸ organisms/mL. Ethylene/maleic anhydride copolymer (for example, as produced by Monsanto, St. Louis, Missouri) is then added to a concentration of 3% v/v. The pH is then adjusted with 1N NaOH to between pH 6.8 and pH 7.7. Finally, NEOCRYL A640∼ is added to a concentration of 1% w/v.

### Example 3 - Serum Neutralization Assay

The serum neutralization assay is conducted as follows:
(a) Serum samples are filter sterilized and the complement in the samples is inactivated by heating the serum samples to 56'C for 30 minutes.
(b) Serial dilutions of the serum are made using PBS as the diluent. The dilutions are added to separate tubes.
(c) Actively growing B. burgdorferi (0.4 mL, 3 x 10⁴ organisms/mL) were added to each tube and gently mixed with the serum samples.
(d) Filter-sterilized guinea pig complement (0.8 mL, obtained from Sigma, St. Louis, MO) was added to each tube and the contents were gently mixed.
(e) The tubes were incubated at 35'C with agitation for one hour.
(f) A 0.1 mL aliquot from each tube was separately added to 0.9 mL of BSK II medium in a culture tube. The cultures were grown at 35'C for seven days.

At the end of the seven day incubation, the cultures were scored for the number of viable bacteria using darkfield microscopy. The "serum neutralizing titer" was scored as the highest dilution of serum which decreased the spirochete count by 80% relative to pre-immune serum.

The present inventors have observed that whenever the serum neutralizing titer of an immunized dog is greater than 4, the dog remains effectively protected from spirochetemia induced by challenge with isolated B. burgdorferi spirochetes.

The serum neutralization assay has also been used to shown that H3TS, a monoclonal antibody against Osp A (obtained from Russell C. Johnson at the University of Minnesota), is effective in the serum neutralization assay (titer=1:236). Monoclonal antibody Cb-2 against Osp B is also effective in neutralizing B. burgdorferi by this assay. A monoclonal antibody against the flagellar protein (41K) was not effective.

### Example 4 - Immunization

### Vaccinations

Dogs were divided between a vaccinated group and a non-vaccinated control group. The vaccinated group received two 1 mL intramuscular injections of the vaccine of Example 2. The second injection followed the first by 22 days. Some of the data will be expressed in terms of the number of assays post 1st vaccination ("dpv")or number of days post 2nd vaccination ("dpv-2").

Practitioners will readily appreciate that the precise conditions used to inactivate intrinsic complement, i.e. the dilution buffer, the number of organisms added, the amount of complement added, the source of complement, the incubation conditions following the addition of complement, and the conditions used in the final incubation are not critical and may be varied as desired or as particular circumstances dictate.

### B. burgdorferi Challenge

At 156 days post 2nd vaccination all of the dogs were challenged with B. burgdorferi using the method described in Example 1. The challenge regimen included seven consecutive daily injections of 5000 spirochetes and a 2mg injection of dexamethasone on the first day of challenge and on the sixth day following initiation of the challenge (day post challenge or "dpc").

### Results

Table 6 shows that the vaccine was effective in protecting dogs from spirochetemia from a B. burgdorferi spirochete challenge initiated 5 months after vaccination. By unpaired student-t test, the statistical significance of the difference is p=0.006.

Table 7 shows that the vaccine was effective in protecting dogs from spirochete-induced arthritis as manifested by limping behavior. By Chi-squared analysis, the statistical significance of the difference between the group is p < 0.01.

Table 8 shows that the vaccine was effective in limiting fevers observed after challenge with B. burgdorferi. Not only are fevers observed more infrequently for vaccinated dogs, the fevers are not observed after 41 days post challenge. In contrast, unvaccinated dogs develope fevers over a longer time span (up to 60 days post challenge). By unpaired student-t test the statistical significance of the difference in these observations over the 38 days of observation was p < 0.01.

Table 9 shows that the vaccinated dogs retained a serum neutralizing titer of anti B. burgdorferi antibodies at 156 days post 2nd vaccination. Spirochete challenge was effective in boosting this titer in the vaccinated dogs. In contrast, the unvaccinated dogs mounted a weak antibody response.

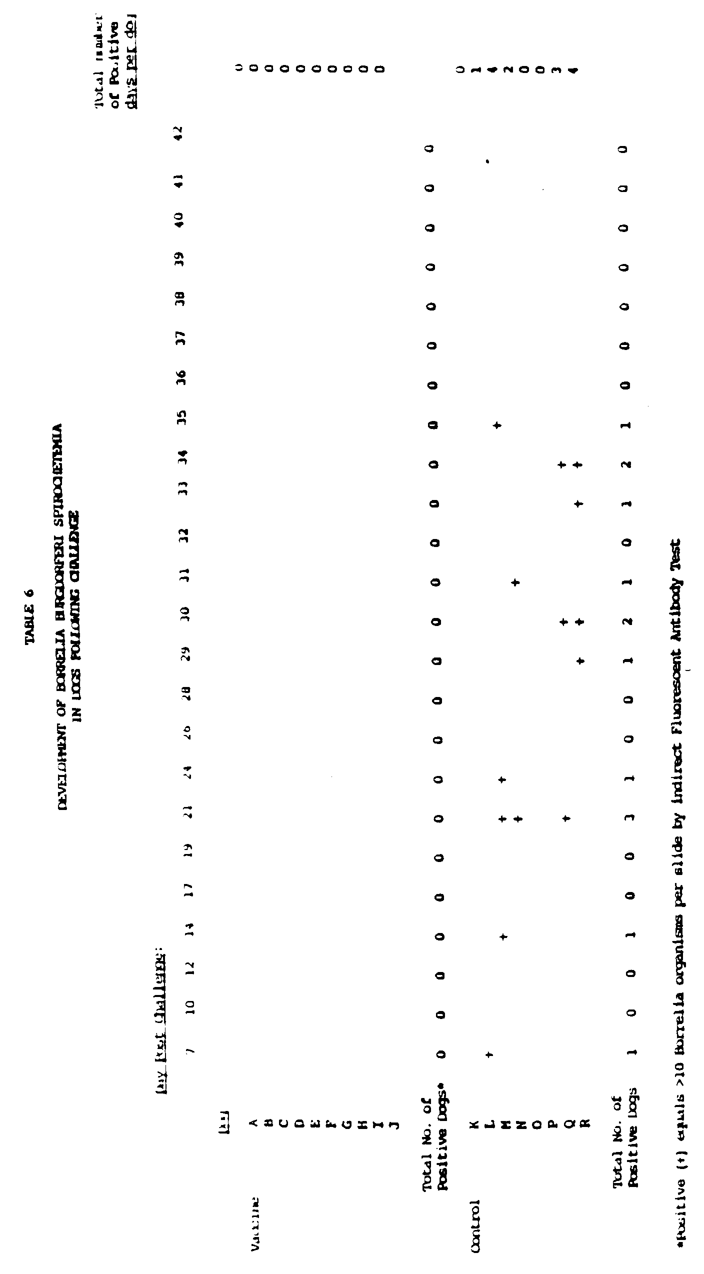

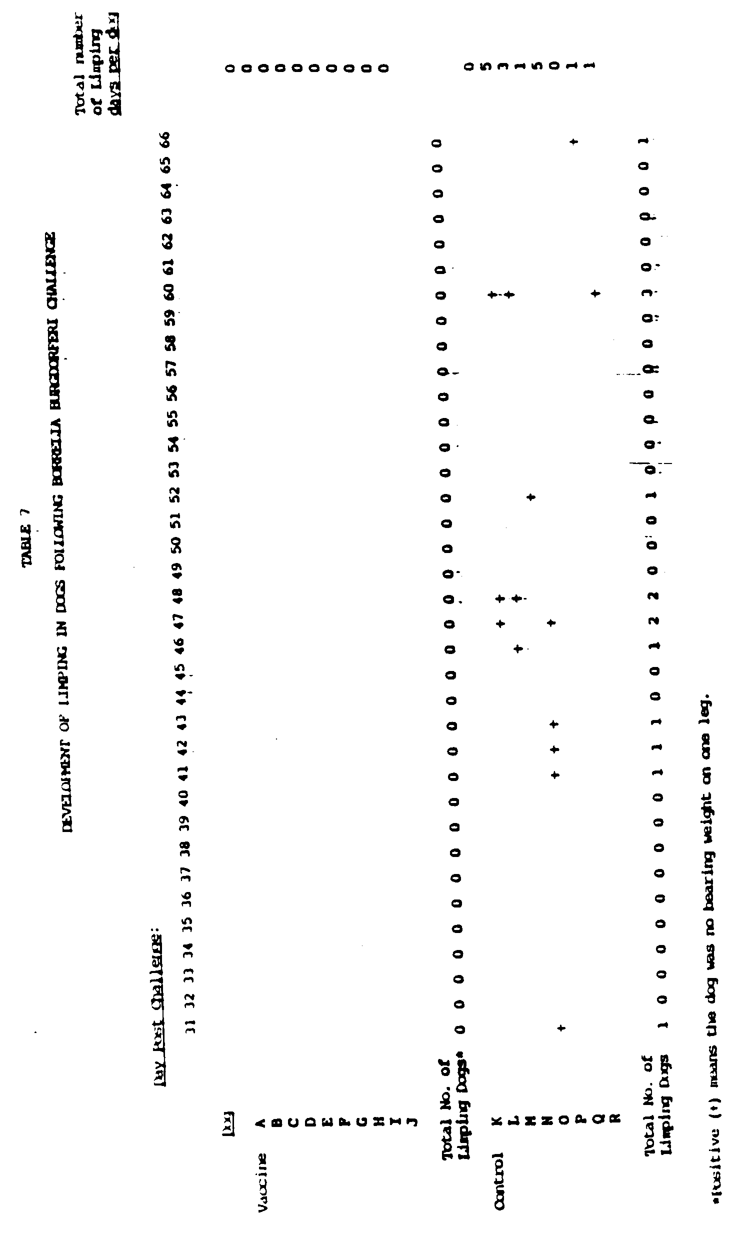

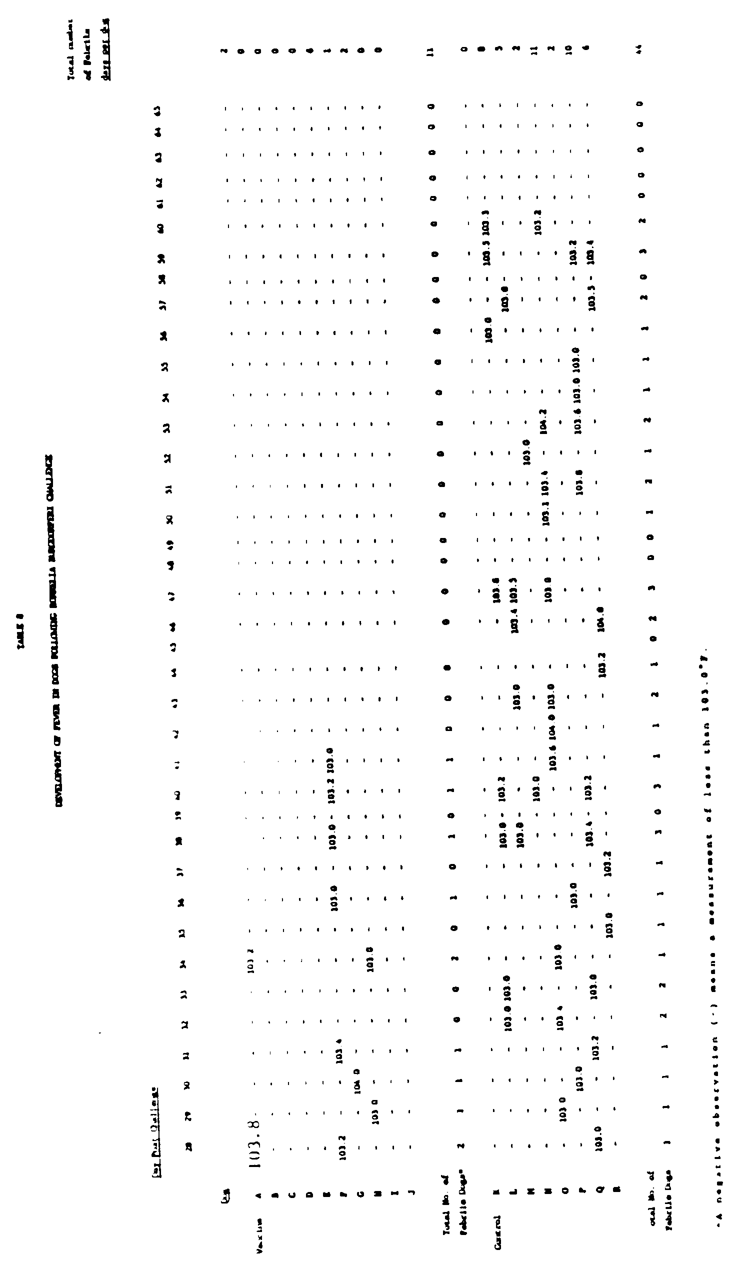

**TABLE 9**

| SERUM NEUTRALIZATION ANTIBODY TITER AGAINST BORRELIA BURGDORFERI | | | | |
|---|---|---|---|---|
| Dog | O DPV | 7 DPV-2 | 156 DPV-2 O DPC | 21 DPC |
| A | <4 | 2048 | 23 | 1024 |
| B | <4 | 4096 | 128 | 1024 |
| C | <4 | 1024 | 16 | 512 |
| D | <4 | 4096 | 32 | 1024 |
| E | <4 | 4096 | 8 | 512 |
| F | <4 | 2048 | 64 | 1024 |
| G | <4 | 4096 | 128 | 1024 |
| H | <4 | 1024 | 64 | 2048 |
| I | <4 | 1024 | 16 | 1024 |
| J | <4 | 4096 | 32 | 512 |
| GMT¹ | <4 | 2291 | 36 | 871 |
| K | ND | ND | <4 | <4 |
| L | ND | ND | <4 | 4 |
| M | ND | ND | <4 | 8 |
| N | ND | ND | <4 | 8 |
| O | ND | ND | <4 | 16 |
| P | ND | ND | <4 | <4 |
| Q | ND | ND | <4 | 4 |
| R | ND | ND | <4 | 4 |
| GMT | | | <4 | 4 |

### Example - 5 Western Blotting

Established techniques were used to conduct a Western blot of B. burgdorferi proteins using serum from vaccinated dogs to identify major antigens. Western blotting techniques are described in Molecular Cloning 2nd edition (supra) on pp. 18.47-18.75.

Figure 1 shows a representation of Western blots of B. burgdorferi visualized with antisera obtained at 154 days post 2nd vaccination from dogs A and B (vaccinated) and dogs K and L (unvaccinated).

The major B. burgdorferi antigens identified by antisera which is effective in a serum neutralization assay have molecular weights (±3K) of 14, 17, 19, 25, 28, 31, 34, 38, 41.44. 48. 52, 54, 58, 60, 68, 80 and 90K.

## Claims (Claims for the following Contracting State(s): AT, BE, CH, DE, DK, FR, GB, IT, LI, LU, NL, SE)

1. A vaccine for protecting a susceptible mammal from Lyme disease comprising an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) such that each dose comprises antigen derived from about 10⁶ to about 10¹² spirochetes.

2. A vaccine as claimed in Claim 1 wherein said vaccine is effective to protect a susceptible mammal from Lyme disease caused by variant strains of B burgdorferi.

3. A vaccine as claimed in Claim 1 or Claim 2 wherein said mammal is one of feline, canine, avian, equine, ovine and bovine.

4. A vaccine as claimed in any one of Claims 1 - 3, wherein said mammal is man.

5. A vaccine as claimed in any one of claims 1 to 4 for protecting a susceptible mammal from Lyme disease comprising an amount of B. burgdorferi major antigen effective to protect a susceptible mammal from at least one of arthritis and fever due to Lyme disease.

6. A vaccine as claimed in any one of Claims 1 - 5 further comprising at least one vaccine selected from the group consisting of vaccines against Leptospira canicola, Leptospira icterohaemorrhagia, Leptospira Leptospira hardjo, Leptospira grippotyphasa, feline leukemia, rhinotracheitis, calici, panleukopenia, chlamydia psittaci, canine distemper, adenovirus Type 2, coronavirus, parainfluenza and parvovirus.

7. A process for preparing a vaccine against Lyme disease comprising an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) such that each dose comprises antigen derived from about 10⁶ to about 10¹² spirochetes, said process comprising:
culturing B. burgdorferi bacteria,
monitoring the level of one of Osp A or Osp B,
inactivating the bacteria when a maxiumum in the production of one of Osp A or Osp B is obtained, and;
extracting the major antigens of B burgdorferi with a surfactant.

8. A process as claimed in Claim 7 further comprising monitoring the bacteria for the percent which express one of Osp A or Osp B.

9. A process as claimed in Claim 8 further requiring that at least 70% of the cultured bacteria express one of Osp A or Osp B.

10. A process as claimed in Claim 9 wherein at least 90% of said cultured bacteria express one of Osp A or Osp B.

11. A process as claimed in any one of Claims 7 - 10 wherein said inactivation comprises adding an amount of glutaraldehyde or formaldehyde effective to inactivate B. burgdorferi.

12. A process as claimed in Claim 11 wherein the amount of aldehyde added is about 0.05% to about 1.0% v/v.

13. A process as claimed in any one of Claims 7 - 12 further comprising adding an adjuvant to said antigens.

14. A process as claimed in Claim 13 further comprising adding at least one more adjuvant to said antigens.

15. A process as claimed in Claim 14 wherein said adjuvants are selected from saponin, oil, ethylene / maleic anhydride copolymer and acrylic acid/copolymers of acrylic acid and methacrylic acid and styrene (Neocryl).

16. A process as claimed in Claim 15 wherein said adjuvants are ethylene / maleic anyydride copolymer and acrylic acid/copolymers of acrylic acid and methacrylic acid and styrene (Neocryl).

17. A process as claimed in any one of Claim 7 - 16 comprising
terminating said culturing when a maximum in the production as one of Osp A or Osp B is obtained,
extracting the major antigens from said bacteria, and
mixing said extracted antigens with a physiologically acceptable carrier.

18. A vaccine for protecting a susceptible mammal from Lyme disease comprising an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) such that each dose comprises antigen derived from about 10⁶ to about 10¹² spirochetes, said vaccine being obtainable by a process comprising the steps of:
culturing B. burgdorferi bacteria,
monitoring the level of one of Osp A or Osp B,
inactivating the bacteria when a maxiumum in the production of one of Osp A or Osp B is obtained; and
extracting the said major antigens of B burgdorferi with a surfactant selected from SDS and octylphenoxy polyethoxyethanol (available under the trade name 'Triton X-100').

19. A vaccine as claimed in Claim 18 wherein said vaccine is effective to protect a susceptible mammal from Lyme disease caused by variant strains of B burgdorferi.

20. A vaccine as claimed in Claim 18 or Claim 19 wherein said mammal is one of feline, canine, avian, equine, ovine and bovine.

21. A vaccine as claimed in any one of Claims 18 to 20, wherein said mammal is man.

22. A vaccine as claimed in any one of claims 18 to 21 for protecting a susceptible mammal from Lyme disease comprising an amount of B. burgdorferi major antigen effective to protect a susceptible mammal from at least one of arthritis and fever due to Lyme disease.

23. A vaccine as claimed in any one of Claims 18 to 22 further comprising at least one vaccine selected from the group consisting of vaccines against Leptospira canicola, Leptospira icterohaemorrhagia, Leptospira hardjo, Leptospira grippotyphasa, feline leukemia, rhinotracheitis, calici, panleukopenia, chlamydia psittaci, canine distemper, adenovirus Type 2, coronavirus, parainfluenza and parvovirus.

24. A vaccine as claimed in any one of Claims 18 to 23 wherin the said process further comprises monitoring the bacteria for the percent which express one of Osp A or Osp B.

25. A vaccine as claimed in any one of Claims 18 to 24 wherein the said process further comprises requiring that at least 70% of the cultured bacteria express one of Osp A or Osp B.

26. A vaccine as claimedin any one of claims 18 to 25 wherein at least 90% of said cultured bacteria express one of Osp A or Osp B.

27. A vaccine as claimed in any one of Claims 18 to 26 wherein the inactivation step comprises adding an amount of glutaraldehyde or formaldehyde effective to inactivate B. burgdorferi.

28. A vaccine as claimed in Claim 27 wherein the amount of aldehyde added is about 0.05% to about 1.0% v/v.

29. A vaccine as claimed in any one of Claims 18 to 28 further comprising an adjuvant

30. A vaccine as claimed in Claim 29 further comprising at least one more adjuvant.

31. A vaccine as claimed in Claim 29 or Claim 30 wherein said adjuvants are selected from saponin, oil, ethylene / maleic anhydride copolymer and acrylic acid/copolymers of acrylic acid and methacrylic acid and styrene (Neocryl).

32. A vaccine as claimed in Claim 31 wherein said adjuvants are ethylene / maleic anyydride copolymer and acrylic acid/copolymers of acrylic acid and methacrylic acid and styrene (Neocryl).

33. A process for preparing a vaccine comprising combining an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) with a physiologically acceptable carrier such that each dose comprises antigen derived from about 10⁶ to about 10¹² spirochetes.

34. A process for preparing a vaccine comprising combining an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) with an adjuvant such that each dose comprises antigen derived from about 10⁶ to about 10¹² spirochetes.

## Claims (Claims for the following Contracting State(s): ES, GR)

1. A process for preparing a vaccine for protecting a susceptible mammal from Lyme disease comprising an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K
antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) such that each dose comprises antigen derived from about 10⁶ to about 10¹² spirochetes which process comprises:
culturing B. burgdorferi bacteria,
monitoring the level of one of Osp A or Osp B,
inactivating the bacteria when a maxiumum in the production of one of Osp A or Osp B is obtained, and;
extracting the major antigens of B burgdorferi with a surfactant.

2. A process as claimed in Claim 1 wherein said vaccine is effective to protect a susceptible mammal from Lyme disease caused by variant strains of B burgdorferi.

3. A process as claimed in Claim 2 wherein said mammal is one of feline, canine, avian, equine, ovine and bovine.

4. A process as claimed in Claim 2 wherein said mammal is man.

5. A process as claimed in any one of claims 1 to 4 wherein said vaccine is for protecting a susceptible mammal from Lyme disease comprising an amount of B. burgdorferi major antigen effective to protect a susceptible mammal from at least one of arthritis and fever due to Lyme disease.

6. A process as claimed in any one of Claims 1 - 5 wherein said vaccine further comprises at least one vaccine selected from the group consisting of vaccines against Leptospira canicola, Leptospira icterohaemorrhagia, Leptospira hardjo, Leptospira grippotyphasa, feline leukemia, rhinotracheitis, calici, panleukopenia, chlamydia psittaci, canine distemper, adenovirus Type 2, coronavirus, parainfluenza and parvovirus.

7. A process as claimed in Claim any one of claims 1 to 6 further comprising monitoring the bacteria for the percent which express one of Osp A or Osp B.

8. A process as claimed in Claim 7 further requiring that at least 70% of the cultured bacteria express one of Osp A or Osp B.

9. A process as claimed in Claim 8 wherein at least 90% of said cultured bacteria express one of Osp A or Osp B.

10. A process as claimed in any one of the preceding claims wherein said inactivation comprises adding an amount of glutaraldehyde or formaldehyde effective to inactivate B. burgdorferi.

11. A process as claimed in Claim 10 wherein the amount of aldehyde added is about 0.05% to about 1.0% v/v.

12. A process as claimed in any one of the preceding claims further comprising adding an adjuvant to said antigens.

13. A process as claimed in Claim 12 further comprising adding at least one more adjuvant to said antigens.

14. A process as claimed in Claim 13 wherein said adjuvants are selected from saponin, oil, ethylene / maleic anhydride copolymer and acrylic acid/copolymers of acrylic acid and methacrylic acid and styrene (Neocryl).

15. A process as claimed in Claim 14 wherein said adjuvants are ethylene / maleic anyydride copolymer and acrylic acid/copolymers of acrylic acid and methacrylic acid and styrene (Neocryl).

16. A process as claimed in any one of the preceding claims comprising:
terminating said culturing when a maximum in the production as one of Osp A or Osp B is obtained,
extracting the major antigens from said bacteria, and
mixing said extracted antigens with a physiologically acceptable carrier.

17. A process for preparing a vaccine comprising combining an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) with a physiologically acceptable carrier such that each dose comprises antigen derived from about 10⁶ to about 10¹² spirochetes.

18. A process for preparing a vaccine comprising combining an amount of B. burgdorferi major antigens effective to induce a serum neutralizing titer of antibodies against B burgdorferi wherein said major antigens are selected from the 25K, 28K, 31K, 34K, 38K, 41K,44K, 48K, 52K, 54K, 58K, 60K, 68K, 80K, and 90K antigens of B. burgdorferi, wherein the molecular weight of each such antigen may vary within ±3K wherein said vaccine comprises at least two of the major antigens of B. burgdorferi wherein at least one of said at least two major antigens is Osp A (31 kDa) or Osp B (34 kDa) with an adjuvant such that each dose comprises antigen derived from about 10⁶ to about 10¹² spirochetes.

## Patentansprüche (Patentansprüche für folgende(n) Vertragsstaat(en): AT, BE, CH, DE, DK, FR, GB, IT, LI, LU, NL, SE)

1. Vakzin zum Schutz eines Säugers, der für die Lyme-Krankheit anfällig ist, umfassend eine Menge an B. burgdorferi-Hauptantigenen, die wirksam ist, um einen Serum-neutralisierenden Titer von Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, so dass jede Dosis Antigen enthält, welches von etwa 10⁶ bis etwa 10¹² Spirochaeten stammt.

2. Vakzin nach Anspruch 1, wobei das Vakzin wirksam ist, um einen dafür anfälligen Säuger gegen Lyme-Krankheit zu schützen, die von Stammvarianten von B. burgdorferi verursacht ist.

3. Vakzin nach Anspruch 1 oder 2, wobei der Säuger eines von Katze, Hund, Vogel, Pferd, Schaf und Rind ist.

4. Vakzin nach einem der Ansprüche 1 bis 3, wobei der Säuger ein Mensch ist.

5. Vakzin nach einem der Ansprüche 1 bis 4 zum Schutz eines dafür anfälligen Säugers gegen Lyme-Krankheit, umfassend eine Menge des B. burgdorferi-Hauptantigens, die wirksam ist, um einen dafür anfälligen Säuger gegen mindestens eines von Arthritis und Fieber infolge von Lyme-Krankheit zu schützen.

6. Vakzin nach einem der Ansprüche 1 bis 5, welches weiters mindestens ein Vakzin ausgewählt aus der Gruppe bestehend aus Vakzinen gegen Leptospira canicola, Leptospira icterohaemorrhagia, Leptospira hardjo, Leptospira grippotyphasa, Katzenleukämie, Rhinotracheitis calici, Panleukopänie, Chlamydia psittaci, Hundestaupe, Adenovirus Typ 2, Coronavirus, Parainfluenza und Parvovirus umfasst.

7. Verfahren zur Herstellung eines Vakzins gegen Lyme-Krankheit, umfassend eine Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, so dass jede Dosis Antigen enthält, welches von etwa 10⁶ bis etwa 10¹² Spirochaeten stammt, welches Verfahren umfasst:
Züchten von B. burgdorferi-Bakterien,
Überwachen der Menge von einem von Osp A oder Osp B,
Inaktivieren der Bakterien, wenn ein Produktionsmaximum eines von Osp A oder Osp B erhalten ist, und
Extrahieren der Hauptantigene von B. burgdorferi mit einem grenzflächenaktiven Mittel.

8. Verfahren nach Anspruch 7, welches weiters das Überwachen der Bakterien auf die Prozent, welche eines von Osp A oder Osp B exprimieren, umfasst.

9. Verfahren nach Anspruch 8, bei welchem es weiters notwendig ist, dass zumindest 70% der gezüchteten Bakterien eines von Osp A oder Osp B exprimieren.

10. Verfahren nach Anspruch 9, wobei mindestens 90% der gezüchteten Bakterien eines von Osp A oder Osp B exprimieren.

11. Verfahren nach einem der Ansprüche 7 bis 10, wobei die Inaktivierung die Zugabe einer Menge an Glutaraldehyd oder Formaldehyd, die zur Inaktivierung von B. burgdorferi wirksam ist, umfasst.

12. Verfahren nach Anspruch 11, wobei die zugesetzte Menge an Aldehyd etwa 0,05% bis etwa 1,0% V/V ist.

13. Verfahren nach einem der Ansprüche 7 bis 12, welches weiters die Zugabe eines Adjuvans zu den Antigenen umfasst.

14. Verfahren nach Anspruch 13, welches weiters die Zugabe mindestens eines weiteren Adjuvans zu den Antigenen umfasst.

15. Verfahren nach Anspruch 14, wobei die Adjuvantien ausgewählt sind aus Saponin, Öl, Ethylen/Maleinsäureanhydrid-Copolymer und Acrylsäure/Copolymeren von Acrylsäure und Methacrylsäure und Styrol (Neocryl).

16. Verfahren nach Anspruch 15, wobei die Adjuvantien Ethylen/Maleinsäureanhydrid-Copolymer und Acrylsäure/Copolymeren von Acrylsäure und Methacrylsäure und Styrol (Neocryl) sind.

17. Verfahren nach einem der Ansprüche 7 bis 16, umfassend
Beenden der Züchtung, wenn ein Produktionsmaximum eines von Osp A oder Osp B erreicht ist,
Extrahieren der Hauptantigene aus diesen Bakterien, und
Mischen der extrahierten Antigene mit einem physiologisch akzeptablen Träger.

18. Vakzin zum Schutz eines dafür anfälligen Säugers gegen Lyme-Krankheit, umfassend eine Menge an B. burgdorferi-Hauptantigenen, die wirksam ist, um einen Serum-neutralisierenden Titer von Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, so dass jede Dosis Antigen enthält, welches von etwa 10⁶ bis etwa 10¹² Spirochaeten stammt, wobei das Vakzin mittels eines Verfahrens erhältlich ist, welches die Schritte umfasst:
Züchten von B. burgdorferi-Bakterien,
Überwachen der Menge von einem von Osp A oder Osp B,
Inaktivieren der Bakterien, wenn ein Produktionsmaximum eines von Osp A oder Osp B erhalten ist, und
Extrahieren der Hauptantigene von B. burgdorferi mit einem grenzflächenaktiven Mittel ausgewählt aus SDS und Octylphenoxypolyethoxyethanol (erhältlich unter der Handelsbezeichnung 'Triton X-100').

19. Vakzin nach Anspruch 18, wobei das Vakzin wirksam ist, um einen dafür anfälligen Säuger gegen Lyme-Krankheit zu schützen, die von Stammvarianten von B. burgdorferi verursacht ist.

20. Vakzin nach Anspruch 18 oder Anspruch 19, wobei der Säuger eines von Katze, Hund, Vogel, Pferd, Schaf und Rind ist.

21. Vakzin nach einem der Ansprüche 18 bis 20, wobei der Säuger ein Mensch ist.

22. Vakzin nach einem der Ansprüche 18 bis 21 zum Schutz eines dafür anfälligen Säugers gegen Lyme-Krankheit, umfassend eine Menge des B. burgdorferi-Hauptantigens, die wirksam ist, um einen dafür anfälligen Säuger gegen mindestens eines von Arthritis und Fieber infolge von Lyme-Krankheit zu schützen.

23. Vakzin nach einem der Ansprüche 18 bis 22, welches weiters mindestens ein Vakzin ausgewählt aus der Gruppe bestehend aus Vakzinen gegen Leptospira canicola, Leptospira icterohaemorrhagia, Leptospira hardjo, Leptospira grippotyphasa, Katzenleukämie, Rhinotracheitis calici, Panleukopänie, Chlamydia psittaci, Hundestaupe, Adenovirus Typ 2, Coronavirus, Parainfluenza und Parvovirus umfasst.

24. Vakzin nach einem der Ansprüche 18 bis 23, wobei das Verfahren weiters das Überwachen der Bakterien auf die Prozent, welche eines von Osp A oder Osp B exprimieren, umfasst.

25. Vakzin nach einem der Ansprüche 18 bis 24, wobei das Verfahren weiters die Notwendigkeit umfasst, dass zumindest 70% der gezüchteten Bakterien eines von Osp A oder Osp B exprimieren.

26. Vakzin nach Anspruch 18 bis 25, wobei mindestens 90% der gezüchteten Bakterien eines von Osp A oder Osp B exprimieren.

27. Vakzin nach einem der Ansprüche 18 bis 26, wobei der Inaktivierungsschritt die Zugabe einer Menge an Glutaraldehyd oder Formaldehyd, die zur Inaktivierung von B. burgdorferi wirksam ist, umfasst.

28. Vakzin nach Anspruch 27, wobei die zugesetzte Menge an Aldehyd etwa 0,05% bis etwa 1,0% V/V ist.

29. Vakzin nach einem der Ansprüche 18 bis 28, welches weiters ein Adjuvans umfasst.

30. Vakzin nach Anspruch 29, welches weiters mindestens ein weiteres Adjuvans umfasst.

31. Vakzin nach Anspruch 29 oder Anspruch 30, wobei die Adjuvantien ausgewählt sind aus Saponin, Öl, Ethylen/Maleinsäurean hydrid-Copolymer und Acrylsäure/Copolymeren von Acrylsäure und Methacrylsäure und Styrol (Neocryl).

32. Vakzin nach Anspruch 31, wobei die Adjuvantien Ethylen/ Maleinsäureanhydrid-Copolymer und Acrylsäure/Copolymeren von Acrylsäure und Methacrylsäure und Styrol (Neocryl) sind.

33. Verfahren zur Herstellung eines Vakzins, umfassend das Vereinigen einer Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, mit einem physiologisch akzeptablen Träger, so dass jede Dosis Antigen enthält, das von etwa 10⁶ bis etwa 10¹² Spirochaeten stammt.

34. Verfahren zur Herstellung eines Vakzins, umfassend das Vereinigen einer Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, mit einem Adjuvans, so dass jede Dosis Antigen enthält, das von etwa 10⁶ bis etwa 10¹² Spirochaeten stammt.

## Patentansprüche (Patentansprüche für folgende(n) Vertragsstaat(en): ES, GR)

1. Verfahren zur Herstellung eines Vakzins zum Schutz eines dafür anfälligen Säugers gegen Lyme-Krankheit, umfassend eine Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, so dass jede Dosis Antigen enthält, das von etwa 10⁶ bis etwa 10¹² Spirochaeten stammt, welches Verfahren umfasst:
Züchten von B. burgdorferi-Bakterien,
Überwachen der Menge von einem von Osp A oder Osp B,
Inaktivieren der Bakterien, wenn ein Produktionsmaximum eines von Osp A oder Osp B erhalten ist, und
Extrahieren der Hauptantigene von B. burgdorferi mit einem grenzflächenaktiven Mittel.

2. Verfahren nach Anspruch 1, wobei das Vakzin wirksam ist, um einen dafür anfälligen Säuger gegen Lyme-Krankheit zu schützen, die von Stammvarianten von B. burgdorferi verursacht ist.

3. Verfahren nach Anspruch 2, wobei der Säuger eines von Katze, Hund, Vogel, Pferd, Schaf und Rind ist.

4. Verfahren nach Anspruch 2, wobei der Säuger ein Mensch ist.

5. Verfahren nach einem der Ansprüche 1 bis 4, wobei das Vakzin zum Schutz eines dafür anfälligen Säugers gegen Lyme-Krankheit vorgesehen ist und eine Menge des B. burgdorferi-Hauptantigens aufweist, die wirksam ist, um einen dafür anfälligen Säuger gegen mindestens eines von Arthritis und Fieber infolge von Lyme-Krankheit zu schützen.

6. Verfahren nach einem der Ansprüche 1 bis 5, wobei das Vakzin weiters mindestens ein Vakzin ausgewählt aus der Gruppe bestehend aus Vakzinen gegen Leptospira canicola, Leptospira icterohaemorrhagia, Leptospira hardjo, Leptospira grippotyphasa, Katzenleukämie, Rhinotracheitis calici, Panleukopänie, Chlamydia psittaci, Hundestaupe, Adenovirus Typ 2, Coronavirus, Parainfluenza und Parvovirus umfasst.

7. Verfahren nach einem der Ansprüche 1 bis 6, welches weiters das Überwachen der Bakterien auf die Prozent, welche eines von Osp A oder Osp B exprimieren, umfasst.

8. Verfahren nach Anspruch 7, bei welchem es weiters notwendig ist, dass zumindest 70% der gezüchteten Bakterien eines von Osp A oder Osp B exprimieren.

9. Verfahren nach Anspruch 8, wobei mindestens 90% der gezüchteten Bakterien eines von Osp A oder Osp B exprimieren.

10. Verfahren nach einem der vorhergehenden Ansprüche, wobei die Inaktivierung die Zugabe einer Menge an Glutaraldehyd oder Formaldehyd, die zur Inaktivierung von B. burgdorferi wirksam ist, umfasst.

11. Verfahren nach Anspruch 10, wobei die zugesetzte Menge an Aldehyd etwa 0,05% bis etwa 1,0% V/V ist.

12. Verfahren nach einem der vorhergehenden Ansprüche, welches weiters die Zugabe eines Adjuvans zu den Antigenen umfasst.

13. Verfahren nach Anspruch 12, welches weiters die Zugabe mindestens eines weiteren Adjuvans zu den Antigenen umfasst.

14. Verfahren nach Anspruch 13, wobei die Adjuvantien ausgewählt sind aus Saponin, Öl, Ethylen/Maleinsäureanhydrid-Copolymer und Acrylsäure/Copolymeren von Acrylsäure und Methacrylsäure und Styrol (Neocryl).

15. Verfahren nach Anspruch 14, wobei diese Adjuvantien Ethylen/Maleinsäureanhydrid-Copolymer und Acrylsäure/Copolymeren von Acrylsäure und Methacrylsäure und Styrol (Neocryl) sind.

16. Verfahren nach einem der vorhergehenden Ansprüche, umfassend
Beenden der Züchtung, wenn ein Produktionsmaximum eines von Osp A oder Osp B erreicht ist,
Extrahieren der Hauptantigene aus diesen Bakterien, und
Mischen der extrahierten Antigene mit einem physiologisch akzeptablen Träger.

17. Verfahren zur Herstellung eines Vakzins, umfassend das Vereinigen einer Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, mit einem physiologisch akzeptablen Träger, so dass jede Dosis Antigen enthält, das von etwa 10⁶ bis etwa 10¹² Spirochaeten stammt.

18. Verfahren zur Herstellung eines Vakzins, umfassend das Vereinigen einer Menge an B. burgdorferi-Hauptantigenen, die wirksam ist, um einen Serum-neutralisierenden Titer an Antikörpern gegen B. burgdorferi zu induzieren, wobei die Hauptantigene ausgewählt sind aus den 25K, 28K, 31K, 34K, 38K, 41K, 44K, 48K, 52K, 54K, 58K, 60K, 68K, 80K und 90K-Antigenen von B. burgdorferi, wobei das Molekulargewicht jedes derartigen Antigens innerhalb von ±3K variieren kann, wobei das Vakzin mindestens zwei der Hauptantigene von B. burgdorferi aufweist, wobei mindestens eines der mindestens zwei Hauptantigene Osp A (31 kDa) oder Osp B (34 kDa) ist, mit einem Adjuvans, so dass jede Dosis Antigen enthält, das von etwa 10⁶ bis etwa 10¹² Spirochaeten stammt.

## Revendications (Revendications pour l'(les) Etat(s) contractant(s) suivant(s): AT, BE, CH, LI, DE, DK, FR, GB, IT, LU, NL, SE)

1. Vaccin pour protéger un mammifère prédisposé de la maladie de Lyme comprenant une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 80K et 90K de B. burgdorferi, dans lequel le poids moléculaire de chaque antigène peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa) tel que chaque dose comprend l'antigène dérivé provenant d'environ 10⁶ a environ 10¹² spirochètes.

2. Vaccin selon la revendication 1 dans lequel ledit vaccin est efficace pour protéger un animal prédisposé de la maladie de Lyme provoquée par des souches variantes de B. burgdorferi.

3. Vaccin selon la revendication 1 ou la revendication 2 dans lequel ledit mammifère est un parmi les races féline, canine, aviaire, chevaline, ovine et bovine.

4. Vaccin selon l'une quelconque des revendications 1-3 dans lequel ledit mammifère est l'homme.

5. Vaccin selon l'une quelconque des revendications 1 à 4 pour protéger un mammifère prédisposé de la maladie de Lyme comprenant une quantité d'antigène majeur de B. burgdorferi efficace pour protéger un mammifère prédisposé d'au moins une parmi l'arthrite ou la fièvre due à la maladie de Lyme.

6. Vaccin selon l'une quelconque des revendications 1-5 comprenant en outre au moins un vaccin choisi parmi le groupe constitué des vaccins contre les Leptospira canicola, Leptospira icterohaemorrhagia, Leptospira hardjo, Leptospira grippotyphasa, la leucémie féline, la rhinotrachéite, calici, la panleucopénie, chlamydia psittaci, la maladie des jeunes chiens, adénovirus de type 2, coronarovirus, parainfluenza et parvovirus.

7. Procédé pour préparer un vaccin contre la maladie de Lyme comprenant une quantité d'antigènes majeurs de B. burgdorferi efficace pour induire un titre de neutralisation du sérum d'anticorps contre B. burgdorferi dans lequel lesdits antigènes majeurs sont choisis parmi les antigènes de 25K, 28K, 31K, 34K, 38K, 41K, 44K, 48K, 52K, 54K, 58K, 60K, 80K et 90K de B. burgdorferi, dans lequel le poids moléculaire de chaque antigène peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa) tel que chaque dose comprend l'antigène dérivé provenant d'environ 10⁶ à environ 10¹² spirochètes, ledit procédé comprenant :
la mise en culture des bactéries B. burgdorferi,
la surveillance du taux de l'une de Osp A ou Osp B,
l'inactivation des bactéries quand un maximum dans la production de Osp A ou Osp B est obtenu, et
l'extraction des antigènes majeurs de B. burgdorferi avec un tensioactif.

8. Procédé selon la revendication 7 comprenant en outre la surveillance des bactéries pour le pourcentage qui exprime une des protéines Osp A ou Osp B.

9. Procédé selon la revendication 8 nécessitant en outre qu'au moins 70% des bactéries cultivées expriment une des protéines Osp A ou Osp B.

10. Procédé selon la revendication 9 dans lequel au moins 90% desdites bactéries cultivées expriment une des protéines Osp A ou Osp B.

11. Procédé selon l'une quelconque des revendications 7-10 dans lequel ladite inactivation comprend l'addition d'une quantité de glutaraldéhyde ou de formaldéhyde efficace pour inactiver B. burgdorferi.

12. Procédé selon la revendication 11 dans lequel la quantité d'aldéhyde ajoutée est environ 0,05% à environ 1,0% v/v.

13. Procédé selon l'une quelconque des revendications 7-12 comprenant en outre l'addition d'un adjuvant auxdits antigènes.

14. Procédé selon la revendication 13 comprenant en outre l'addition d'au moins un ou plusieurs adjuvants auxdits antigènes.

15. Procédé selon la revendication 14 dans lequel lesdits adjuvants sont choisis parmi la saponine, l'huile, un copolymère d'éthylène/anhydride maléique et acide acrylique/copolymères d'acide acrylique et d'acide méthacrylique et de styrène (Néocryl).

16. Procédé selon la revendication 15 dans lequel lesdits adjuvants sont un copolymère d'éthylène/anhydride maléique et acide acrylique/copolymères d'acide acrylique et d'acide méthacrylique et de styrène (Néocryl).

17. Procédé selon l'une quelconque des revendications 7-16 comprenant
l'arrêt de ladite culture quand un maximum dans la production de une des Osp A ou Osp B est obtenu,
l'extraction des antigènes majeurs desdites bactéries, et
le mélange desdits antigènes extraits avec un support physiologique acceptable.

18. Vaccin pour protéger un mammifère prédisposé de la maladie de Lyme comprenant une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène de ce type peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa) tel que chaque dose comprend l'antigène dérivé provenant d'environ 10⁶ à environ 10¹² spirochètes, ledit vaccin pouvant être obtenu par un procédé comprenant les étapes de :
la mise en culture des bactéries B. burgdorferi,
la surveillance du taux de l'une de Osp A ou Osp B,
l'inactivation des bactéries quand un maximum dans la production d'une des Osp A ou Osp B est obtenu, et
l'extraction desdits antigènes majeurs de B. burgdorferi avec un tensioactif choisi parmi SDS et l'octylphénoxypolyéthoxyéthanol (disponible sous la dénomination commerciale 〈〈Triton X-100〉〉).

19. Vaccin selon la revendication 18 dans lequel ledit vaccin est efficace pour protéger un mammifère prédisposé de la maladie de Lyme provoquée par des souches variantes de B. burgdorferi.

20. Vaccin selon la revendication 18 ou la revendication 19 dans lequel ledit mammifère est un parmi les races féline, canine, aviaire, chevaline, ovine et bovine.

21. Vaccin selon l'une quelconque des revendications 18 à 20, dans lequel ledit mammifère est l'homme.

22. Vaccin selon l'une quelconque des revendications 18 à 21 pour protéger un mammifère prédisposé de la maladie de Lyme comprenant une quantité d'antigène majeur de B. burgdorferi efficace pour protéger un mammifère prédisposé d'au moins une parmi l'arthrite ou la fièvre due à la maladie de Lyme.

23. Vaccin selon l'une quelconque des revendications 18 à 22 comprenant en outre au moins un vaccin choisi parmi le groupe constitué des vaccins contre les Leptospira canicola, Leptospira icterohaemorrhagia, Leptospira hardjo, Leptospira grippotyphasa, la leucémie féline, la rhinotrachéite, calici, la panleucopénie, chlamydia psittaci, la maladie des jeunes chiens, adénovirus de type 2, coronarovirus, parainfluenza et parvovirus.

24. Vaccin selon l'une quelconque des revendications 18 à 23 dans lequel ledit procédé comprend en outre la surveillance des bactéries pour le pourcentage qui exprime une des protéines Osp A ou Osp B.

25. Vaccin selon l'une quelconque des revendications 18 à 24 dans lequel ledit procédé comprend en outre la nécessité qu'au moins 70% des bactéries cultivées expriment une des protéines Osp A ou Osp B.

26. Vaccin selon l'une quelconque des revendications 18 à 25 dans lequel au moins 90% desdites bactéries cultivées expriment une des protéines Osp A ou Osp B.

27. Vaccin selon l'une quelconque des revendications 18 à 26 dans lequel l'étape d'inactivation comprend l'addition d'une quantité de glutaraldéhyde ou de formaldéhyde efficace pour inactiver B. burgdorferi.

28. Vaccin selon la revendication 27 dans lequel la quantité d'aldéhyde ajoutée est environ 0,05% à environ 1,0% v/v.

29. Vaccin selon l'une quelconque des revendications 18 à 28 comprenant en outre un adjuvant.

30. Vaccin selon la revendication 29 comprenant au moins un ou plusieurs adjuvants.

31. Vaccin selon la revendication 29 ou la revendication 30 dans lequel lesdits adjuvants sont choisis parmi la saponine, l'huile, un copolymère d'éthylène/anhydride maléique et acide acrylique/copolymères d'acide acrylique et d'acide méthacrylique et de styrène (Néocryl).

32. Vaccin selon la revendication 31 dans lequel lesdits adjuvants sont un copolymère d'éthylène/anhydride maléique et acide acrylique/copolymères d'acide acrylique et d'acide méthacrylique et de styrène (Néocryl).

33. Procédé pour préparer un vaccin comprenant la combinaison d'une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa) avec un support physiologiquement acceptable tel que chaque dose comprend l'antigène dérivé provenant d'environ 10⁶ a environ 10¹² spirochètes.

34. Procédé pour préparer un vaccin comprenant la combinaison d'une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène de ce type peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa) avec un adjuvant tel que chaque dose comprend l'antigène dérivé provenant d'environ 10⁶ a environ 10¹² spirochètes.

## Revendications (Revendications pour l'(les) Etat(s) contractant(s) suivant(s): ES, GR)

1. Procédé pour préparer un vaccin pour protéger un mammifère prédisposé de la maladie de Lyme comprenant une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène de ce type peut varier dans l'intervalle de ± 3K, dans lequel ledit vaccin comprend au moins 2 des antigènes majeurs de B. burgdorferi dans lequel au moins un desdits au moins 2 antigènes majeurs est Osp A (31 kDa) ou Osp B (34 kDa), tel que chaque dose comprend l'antigène dérivé provenant d'environ 10⁶ a environ 10¹² spirochètes, procédé qui comprend :
la mise en culture des bactéries de B. burgdorferi,
la surveillance du taux de l'une des Osp A ou Osp B,
l'inactivation des bactéries quand un maximum dans la production de l'une des Osp A ou Osp B est obtenu, et
l'extraction des antigènes majeurs de B. burgdorferi avec un tensioactif.

2. Procédé selon la revendication 1 dans lequel ledit vaccin est efficace pour protéger un animal prédisposé de la maladie de Lyme provoquée par des souches variantes de B. burgdorferi.

3. Procédé selon la revendication 2 dans lequel ledit mammifère est un parmi les races féline, canine, aviaire, chevaline, ovine et bovine.

4. Procédé selon la revendication 2 dans lequel ledit mammifère est l'homme.

5. Procédé selon l'une quelconque des revendications 1 à 4 dans lequel ledit vaccin est destiné à protéger un mammifère prédisposé de la maladie de Lyme comprenant une quantité d'antigène majeur de B. burgdorferi efficace pour protéger un mammifère prédisposé d'au moins une parmi l'arthrite ou la fièvre due à la maladie de Lyme.

6. Procédé selon l'une quelconque des revendications 1-5 dans lequel ledit vaccin comprend en outre au moins un vaccin choisi parmi le groupe constitué des vaccins contre les Leptospira canicola, Leptospira icterohaemorrhagia, Leptospira hardjo, Leptospira grippotyphasa, la leucémie féline, la rhinotrachéite, calici, la panleucopénie, chlamydia psittaci, la maladie des jeunes chiens, adénovirus de type 2, coronarovirus, parainfluenza et parvovirus.

7. Procédé selon l'une quelconque des revendications 1 à 6 comprenant en outre la surveillance des bactéries pour le pourcentage qui exprime une des protéines Osp A ou Osp B.

8. Procédé selon la revendication 7 nécessitant en outre qu'au moins 70% des bactéries cultivées expriment une des protéines Osp A ou Osp B.

9. Procédé selon la revendication 8 dans lequel au moins 90% desdites bactéries cultivées expriment une des protéines Osp A ou Osp B.

10. Procédé selon l'une quelconque des revendications précédentes dans lequel ladite inactivation comprend l'addition d'une quantité de glutaraldéhyde ou de formaldéhyde efficace pour inactiver B. burgdorferi.

11. Procédé selon la revendication 10 dans lequel la quantité d'aldéhyde ajoutée est environ 0,05% à environ 1,0% v/v.

12. Procédé selon l'une quelconque des revendications précédentes comprenant en outre l'addition d'un adjuvant auxdits antigènes.

13. Procédé selon la revendication 12 comprenant en outre l'addition d'au moins un ou plusieurs adjuvants auxdits antigènes.

14. Procédé selon la revendication 13 dans lequel lesdits adjuvants sont choisis parmi la saponine, l'huile, un copolymère d'éthylène/anhydride maléique et acide acrylique/copolymères d'acide acrylique et d'acide méthacrylique et de styrène (Néocryl).

15. Procédé selon la revendication 14 dans lequel lesdits adjuvants sont un copolymère d'éthylène/anhydride maléique et acide acrylique/copolymères d'acide acrylique et d'acide méthacrylique et de styrène (Néocryl).

16. Procédé selon l'une quelconque des revendications précédentes comprenant
l'arrêt de ladite culture quand un maximum dans la production d'une des Osp A ou Osp B est obtenu,
l'extraction des antigènes majeurs desdites bactéries, et
le mélange desdits antigènes extraits avec un support physiologique acceptable.

17. Procédé pour préparer un vaccin comprenant la combinaison d'une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène peut varier dans l'intervalle de ± 3K, dans lequel ledit vaccin comprend au moins 2 des antigènes majeurs de B. burgdorferi, dans lequel au moins un desdits au moins 2 antigènes majeurs est Osp A (31 kDa) ou Osp B (34 kDa), avec un support physiologiquement acceptable tel que chaque dose comprend l'antigène dérivé provenant d'environ 10⁶ a environ 10¹² spirochètes.

18. Procédé pour préparer un vaccin comprenant la combinaison d'une quantité d'antigènes majeurs de B. burgdorferi efficace pour induire un titre de neutralisation du sérum d'anticorps contre B. burgdorferi dans lequel lesdits antigènes majeurs sont choisis parmi des antigènes de 25K, 28K, 31K, 34K, 38K, 41K, 44K, 48K, 52K, 54K, 58K, 60K, 68K, 80K et 90K de B. burgdorferi, dans lequel le poids moléculaire de chaque antigène peut varier dans l'intervalle de ± 3K dans lequel ledit vaccin comprend au moins 2 des antigènes majeurs de B. burgdorferi dans lequel au moins un desdits au moins 2 antigènes majeurs est Osp A (31 kDa) ou Osp B (34 kDa) avec un adjuvant tel que chaque dose comprend l'antigène dérivé provenant d'environ 10⁶ a environ 10¹² spirochètes.
